# FASCICULE DE BREVET EUROPEEN

(11) **EP 2 516 676 B1**
(45) Date de publication et mention de la délivrance du brevet: **21.01.2015**
(21) Numéro de dépôt: 10809304.8
(22) Date de dépôt: 21.12.2010
(51) Int. Cl.: C12Q 1/68

(54) **MITF COMME MARQUEUR DE PREDISPOSITION A UN CANCER**
MITF ALS MARKER FÜR KREBSPRÄDISPOSITION
MITF AS A MARKER FOR PREDISPOSITION TO CANCER

(30) Priorité: 21.12.2009 FR 0959325
(43) Date de publication de la demande: 31.10.2012
(73) Titulaire: Institut Gustave Roussy, 94805 Villejuif Cédex (FR); Université Nice Sophia Antipolis, 06108 Nice Cedex 2 (FR); INSERM - Institut National de la Santé et de la Recherche Médicale, 75654 Paris Cedex 13 (FR)
(72) Inventeur: BALLOTTI, Robert, F-06100 Nice (FR); BERTOLOTTO, Corine, F-06100 Nice (FR); BRESSAC DE PAILLERETS, Brigitte, F-92330 Sceaux (FR); DE LICHY, Mahaut, F-75015 Paris (FR); LESUEUR, Fabienne, F-69008 Lyon (FR)
(74) Mandataire: Gallois, Valérie
(86) Numéro de dépôt international: PCT/FR2010/052853
(87) Numéro de publication internationale: WO 2011/083253

(56) Documents cités:
- CRONIN JULIA C ET AL: "Frequent mutations in the MITF pathway in melanoma", PIGMENT CELL & MELANOMA RESEARCH, vol. 22, no. 4, août 2009 (2009-08), pages 435-444, XP002592432,
- YOKOYAMA SATORU ET AL: "MITF pathway mutations in melanoma", PIGMENT CELL & MELANOMA RESEARCH, vol. 22, no. 4, août 2009 (2009-08), pages 376-377, XP002592433,
- MURAKAMI HIDEKI ET AL: "Sumoylation modulates transcriptional activity of MITF in a promoter-specific manner", PIGMENT CELL RESEARCH, vol. 18, no. 4, août 2005 (2005-08), pages 265-277, XP002592434, ISSN: 0893-5785
- MILLER ARLO J ET AL: "Sumoylation of MITF and its related family members TFE3 and TFEB", JOURNAL OF BIOLOGICAL CHEMISTRY, vol. 280, no. 1, 7 janvier 2005 (2005-01-07), pages 146-155, XP002592435, ISSN: 0021-9258
- NIHAL MINAKSHI ET AL: "Anti-proliferative and proapoptotic effects of (-)-epigallocatechin-3-gallate on human melanoma: Possible implications for the chemoprevention of melanoma", INTERNATIONAL JOURNAL OF CANCER, vol. 114, no. 4, avril 2005 (2005-04), pages 513-521, XP002592436, ISSN: 0020-7136

### SEQUENCE LISTING

<110> Institut Gustave Roussy et al
<120> Marqueur de prédisposition à un cancer
<130> B987PC
<160> 40
<170> PatentIn version 3.3
<210> 1
   <211> 20
   <212> DNA
   <213> artificial sequence
<220>
   <223> primer
<400> 1
   cttgaagcaa gtggggagag 20
<210> 2
   <211> 21
   <212> DNA
   <213> artificial sequence
<220>
   <223> primer
<400> 2
   ccactgctgg aaagtgagaa c 21
<210> 3
   <211> 18
   <212> DNA
   <213> artificial sequence
<220>
   <223> primer
<400> 3
   ccgggccgaa ctacagat 18
<210> 4
   <211> 19
   <212> DNA
   <213> artificial sequence
<220>
   <223> primer
<400> 4
   cagagtggca gacgcagtg 19
<210> 5
   <211> 20
   <212> DNA
   <213> artificial sequence
<220>
   <223> primer
<400> 5
   tcctctcctt ttgcttctga 20
<210> 6
   <211> 22
   <212> DNA
   <213> artificial sequence
<220>
   <223> primer
<400> 6
   tgaacaagaa ccaaagattt ca 22
<210> 7
   <211> 20
   <212> DNA
   <213> artificial sequence
<220>
   <223> primer
<400> 7
   cacagccagt gccagaacta 20
<210> 8
   <211> 22
   <212> DNA
   <213> artificial sequence
<220>
   <223> primer
<400> 8
   cccaataaac ccttctcttc ct 22
<210> 9
   <211> 20
   <212> DNA
   <213> Artificial sequence
<220>
   <223> primer
<400> 9
   ggtgcaattt aggatacccc 20
<210> 10
   <211> 21
   <212> DNA
   <213> Artificial sequence
<220>
   <223> primer
<400> 10
   cacctagcaa atggaaaatg g 21
<210> 11
   <211> 20
   <212> DNA
   <213> Artificial sequence
<220>
   <223> primer
<400> 11
   ggcccttatg tgaacgtttt 20
<210> 12
   <211> 22
   <212> DNA
   <213> Artificial sequence
<220>
   <223> primer
<400> 12
   tggcatcaaa taataaacag ca 22
<210> 13
   <211> 20
   <212> DNA
   <213> Artificial sequence
<220>
   <223> primer
<400> 13
   tttgtgcctg aaggaagagc 20
<210> 14
   <211> 20
   <212> DNA
   <213> Artificial sequence
<220>
   <223> primer
<400> 14
   caaaggctgg taaatgtggc 20
<210> 15
   <211> 20
   <212> DNA
   <213> Artificial sequence
<220>
   <223> primer
<400> 15
   gttcatcttg ttgctgtgcc 20
<210> 16
   <211> 23
   <212> DNA
   <213> Artificial sequence
<220>
   <223> primer
<400> 16
   gcttaagttt tcaggaaggt gtg 23
<210> 17
   <211> 21
   <212> DNA
   <213> Artificial sequence
<220>
   <223> primer
<400> 17
   aaagaccatt attgctttgg g 21
<210> 18
   <211> 20
   <212> DNA
   <213> Artificial sequence
<220>
   <223> primer
<400> 18
   aaaagaaccc tggaaacacc 20
<210> 19
   <211> 27
   <212> DNA
   <213> Artificial sequence
<220>
   <223> primer
<400> 19
   ggagatcctg tacctctctt ttaatac 27
<210> 20
   <211> 22
   <212> DNA
   <213> Artificial sequence
<220>
   <223> primer
<400> 20
   tgttttaacc actgcagaga cc 22
<210> 21
   <211> 24
   <212> DNA
   <213> Artificial sequence
<220>
   <223> primer
<400> 21
   caaataagct tctgtatgtt tggg 24
<210> 22
   <211> 22
   <212> DNA
   <213> Artificial sequence
<220>
   <223> primer
<400> 22
   cagctgtagg aatcaactct cc 22
<210> 23
   <211> 20
   <212> DNA
   <213> Artificial sequence
<220>
   <223> primer
<400> 23
   aggttcaggt ttccgttgtc 20
<210> 24
   <211> 20
   <212> DNA
   <213> Artificial sequence
<220>
   <223> primer
<400> 24
   tagaaccaaa gggagagggg 20
<210> 25
   <211> 18
   <212> DNA
   <213> Artificial sequence
<220>
   <223> primer
<400> 25
   tacacggctt gggtggtg 18
<210> 26
   <211> 21
   <212> DNA
   <213> Artificial sequence
<220>
   <223> primer
<400> 26
   catgtccaag aatgactgtg g 21
<210> 27
   <211> 23
   <212> DNA
   <213> artificial sequence
<220>
   <223> primer
<400> 27
   gcttaaaagt cctctgtgct ctg 23
<210> 28
   <211> 22
   <212> DNA
   <213> artificial sequence
<220>
   <223> primer
<400> 28
   caagaaaacc ccttcaggta ag 22
<210> 29
   <211> 16
   <212> DNA
   <213> artificial sequence
<220>
   <223> probe
<400> 29
   atcaagcaag aacccg 16
<210> 30
   <211> 14
   <212> DNA
   <213> artificial sequence
<220>
   <223> probe
<400> 30
   caagcaaaaa cccg 14
<210> 31
   <211> 23
   <212> DNA
   <213> artificial sequence
<220>
   <223> primer
<400> 31
   tgctctccag atttggtgaa tcg 23
<210> 32
   <211> 21
   <212> DNA
   <213> artificial sequence
<220>
   <223> primer
<400> 32
   ggtcttggct gcagttctca a 21
<210> 33
   <211> 29
   <212> DNA
   <213> artificial sequence
<220>
   <223> primer
<400> 33
   cttcccaaca taagaaggga gctcacagc 29
<210> 34
   <211> 29
   <212> DNA
   <213> artificial sequence
<220>
   <223> primer
<400> 34
   ggatcatcaa gcaaaaacca gttcttgag 29
<210> 35
   <211> 520
   <212> PRT
   <213> Homo sapiens
<220>
   <221> SITE
   <222> (289) .. (289)
   <223> Xaa = any amino acid
<220>
   <221> SITE
   <222> (291)..(291)
   <223> Xaa = any amino acid
<220>
   <221> SITE
   <222> (417)..(417)
   <223> Xaa = any amino acid
<220>
   <221> SITE
   <222> (419)..(419)
   <223> Xaa = any amino acid
<400> 35
   Met Gln Ser Glu Ser Gly Ile Val Pro Asp Phe Glu Val Gly Glu Glu
   1               5                   10                  15
   Phe His Glu Glu Pro Lys Thr Tyr Tyr Glu Leu Lys Ser Gln Pro Leu
               20                  25                  30
   Lys Ser Ser Ser Ser Ala Glu His Pro Gly Ala Ser Lys Pro Pro Ile
           35                  40                  45
   Ser Ser Ser Ser Met Thr Ser Arg Ile Leu Leu Arg Gln Gln Leu Met
       50                  55                  60
   Arg Glu Gln Met Gln Glu Gln Glu Arg Arg Glu Gln Gln Gln Lys Leu
   65                  70                  75                  80
   Gln Ala Ala Gln Phe Met Gln Gln Arg Val Pro Val Ser Gln Thr Pro
                   85                  90                  95
   Ala Ile Asn Val Ser Val Pro Thr Thr Leu Pro Ser Ala Thr Gln Val
               100                 105                 110
   Pro Met Glu Val Leu Lys Val Gln Thr His Leu Glu Asn Pro Thr Lys
           115                 120                 125
   Tyr His Ile Gln Gln Ala Gln Arg Gln Gln Val Lys Gln Tyr Leu Ser
       130                 135                 140
   Thr Thr Leu Ala Asn Lys His Ala Asn Gln Val Leu Ser Leu Pro Cys
   145                 150                 155                 160
   Pro Asn Gln Pro Gly Asp His Val Met Pro Pro Val Pro Gly Ser Ser
                   165                 170                 175
   Ala Pro Asn Ser Pro Met Ala Met Leu Thr Leu Asn Ser Asn Cys Glu
               180                 185                 190
   Lys Glu Gly Phe Tyr Lys Phe Glu Glu Gln Asn Arg Ala Glu Ser Glu
           195                 200                 205
   Cys Pro Gly Met Asn Thr His Ser Arg Ala Ser Cys Met Gln Met Asp
       210                 215                 220
   Asp Val Ile Asp Asp Ile Ile Ser Leu Glu Ser Ser Tyr Asn Glu Glu
   225                 230                 235                 240
   Ile Leu Gly Leu Met Asp Pro Ala Leu Gln Met Ala Asn Thr Leu Pro
                   245                 250                 255
   Val Ser Gly Asn Leu Ile Asp Leu Tyr Gly Asn Gln Gly Leu Pro Pro
               260                 265                 270
   Pro Gly Leu Thr Ile Ser Asn Ser Cys Pro Ala Asn Leu Pro Asn Ile
           275                 280                 285
   Xaa Arg Xaa Leu Thr Glu Ser Glu Ala Arg Ala Leu Ala Lys Glu Arg
       290                 295                 300
   Gln Lys Lys Asp Asn His Asn Leu Ile Glu Arg Arg Arg Arg Phe Asn
   305                 310                 315                 320
   Ile Asn Asp Arg Ile Lys Glu Leu Gly Thr Leu Ile Pro Lys Ser Asn
                   325                 330                 335
   Asp Pro Asp Met Arg Trp Asn Lys Gly Thr Ile Leu Lys Ala Ser Val
               340                 345                 350
   Asp Tyr Ile Arg Lys Leu Gln Arg Glu Gln Gln Arg Ala Lys Glu Leu
           355                 360                 365
   Glu Asn Arg Gln Lys Lys Leu Glu His Ala Asn Arg His Leu Leu Leu
       370                 375                 380
   Arg Ile Gln Glu Leu Glu Met Gln Ala Arg Ala His Gly Leu Ser Leu
   385                 390                 395                 400
   Ile Pro Ser Thr Gly Leu Cys Ser Pro Asp Leu Val Asn Arg Ile Ile
                   405                 410                 415
   Xaa Gln Xaa Pro Val Leu Glu Asn Cys Ser Gln Asp Leu Leu Gln His
               420                 425                 430
   His Ala Asp Leu Thr Cys Thr Thr Thr Leu Asp Leu Thr Asp Gly Thr
           435                 440                 445
   Ile Thr Phe Asn Asn Asn Leu Gly Thr Gly Thr Glu Ala Asn Gln Ala
       450                 455                 460
   Tyr Ser Val Pro Thr Lys Met Gly Ser Lys Leu Glu Asp Ile Leu Met
   465                 470                 475                 480
   Asp Asp Thr Leu Ser Pro Val Gly Val Thr Asp Pro Leu Leu Ser Ser
                   485                 490                 495
   Val Ser Pro Gly Ala Ser Lys Thr Ser Ser Arg Arg Ser Ser Met Ser
               500                 505                 510
   Met Glu Glu Thr Glu His Thr Cys
           515                 520
<210> 36
   <211> 504
   <212> PRT
   <213> Homo sapiens
<220>
   <221> SITE
   <222> (273)..(273)
   <223> Xaa = any amino acid
<220>
   <221> SITE
   <222> (275)..(275)
   <223> Xaa = any amino acid
<220>
   <221> SITE
   <222> (401)..(401)
   <223> Xaa = any amino acid
<220>
   <221> SITE
   <222> (403)..(403)
   <223> Xaa = any amino acid
<400> 36
   Met Glu Ala Leu Arg Val Gln Met Phe Met Pro Cys Ser Phe Glu Ser
   1               5                   10                  15
   Leu Tyr Leu Ser Ser Ala Glu His Pro Gly Ala Ser Lys Pro Pro Ile
               20                  25                  30
   Ser Ser Ser Ser Met Thr Ser Arg Ile Leu Leu Arg Gln Gln Leu Met
           35                  40                  45
   Arg Glu Gln Met Gln Glu Gln Glu Arg Arg Glu Gln Gln Gln Lys Leu
       50                  55                  60
   Gln Ala Ala Gln Phe Met Gln Gln Arg Val Pro Val Ser Gln Thr Pro
   65                  70                  75                  80
   Ala Ile Asn Val Ser Val Pro Thr Thr Leu Pro Ser Ala Thr Gln Val
                   85                  90                  95
   Pro Met Glu Val Leu Lys Val Gln Thr His Leu Glu Asn Pro Thr Lys
               100                 105                 110
   Tyr His Ile Gln Gln Ala Gln Arg Gln Gln Val Lys Gln Tyr Leu Ser
           115                 120                 125
   Thr Thr Leu Ala Asn Lys His Ala Asn Gln Val Leu Ser Leu Pro Cys
       130                 135                 140
   Pro Asn Gln Pro Gly Asp His Val Met Pro Pro Val Pro Gly Ser Ser
   145                 150                 155                 160
   Ala Pro Asn Ser Pro Met Ala Met Leu Thr Leu Asn Ser Asn Cys Glu
                   165                 170                 175
   Lys Glu Gly Phe Tyr Lys Phe Glu Glu Gln Asn Arg Ala Glu Ser Glu
               180                 185                 190
   Cys Pro Gly Met Asn Thr His Ser Arg Ala Ser Cys Met Gln Met Asp
           195                 200                 205
   Asp Val Ile Asp Asp Ile Ile Ser Leu Glu Ser Ser Tyr Asn Glu Glu
       210                 215                 220
   Ile Leu Gly Leu Met Asp Pro Ala Leu Gln Met Ala Asn Thr Leu Pro
   225                 230                 235                 240
   Val Ser Gly Asn Leu Ile Asp Leu Tyr Gly Asn Gln Gly Leu Pro Pro
                   245                 250                 255
   Pro Gly Leu Thr Ile Ser Asn Ser Cys Pro Ala Asn Leu Pro Asn Ile
               260                 265                 270
   Xaa Arg Xaa Leu Thr Glu Ser Glu Ala Arg Ala Leu Ala Lys Glu Arg
           275                 280                 285
   Gln Lys Lys Asp Asn His Asn Leu Ile Glu Arg Arg Arg Arg Phe Asn
       290                 295                 300
   Ile Asn Asp Arg Ile Lys Glu Leu Gly Thr Leu Ile Pro Lys Ser Asn
   305                 310                 315                 320
   Asp Pro Asp Met Arg Trp Asn Lys Gly Thr Ile Leu Lys Ala Ser Val
                   325                 330                 335
   Asp Tyr Ile Arg Lys Leu Gln Arg Glu Gln Gln Arg Ala Lys Glu Leu
               340                 345                 350
   Glu Asn Arg Gln Lys Lys Leu Glu His Ala Asn Arg His Leu Leu Leu
           355                 360                 365
   Arg Ile Gln Glu Leu Glu Met Gln Ala Arg Ala His Gly Leu Ser Leu
       370                 375                 380
   Ile Pro Ser Thr Gly Leu Cys Ser Pro Asp Leu Val Asn Arg Ile Ile
   385                 390                 395                 400
   Xaa Gln Xaa Pro Val Leu Glu Asn Cys Ser Gln Asp Leu Leu Gln His
                   405                 410                 415
   His Ala Asp Leu Thr Cys Thr Thr Thr Leu Asp Leu Thr Asp Gly Thr
               420                 425                 430
   Ile Thr Phe Asn Asn Asn Leu Gly Thr Gly Thr Glu Ala Asn Gln Ala
           435                 440                 445
   Tyr Ser Val Pro Thr Lys Met Gly Ser Lys Leu Glu Asp Ile Leu Met
       450                 455                 460
   Asp Asp Thr Leu Ser Pro Val Gly Val Thr Asp Pro Leu Leu Ser Ser
   465                 470                 475                 480
   Val Ser Pro Gly Ala Ser Lys Thr Ser Ser Arg Arg Ser Ser Met Ser
                   485                 490                 495
   Met Glu Glu Thr Glu His Thr Cys
               500
<210> 37
   <211> 519
   <212> PRT
   <213> Homo sapiens
<220>
   <221> SITE
   <222> (288)..(288)
   <223> Xaa = any amino acid
<220>
   <221> SITE
   <222> (290)..(290)
   <223> Xaa = any amino acid
<220>
   <221> SITE
   <222> (416)..(416)
   <223> Xaa = any amino acid
<220>
   <221> SITE
   <222> (418)..(418)
   <223> Xaa = any amino acid
<400> 37
   Met Gly His Leu Glu Asn Thr Ser Val Val Phe Pro Arg Ala Ile Phe
   1               5                   10                  15
   Ser Leu Cys Glu Lys Glu Thr Arg Lys Leu Thr Leu Cys Leu Phe Ser
               20                  25                  30
   Arg Ser Ser Ser Ala Glu His Pro Gly Ala Ser Lys Pro Pro Ile Ser
           35                  40                  45
   Ser Ser Ser Met Thr Ser Arg Ile Leu Leu Arg Gln Gln Leu Met Arg
       50                  55                  60
   Glu Gln Met Gln Glu Gln Glu Arg Arg Glu Gln Gln Gln Lys Leu Gln
   65                  70                  75                  80
   Ala Ala Gln Phe Met Gln Gln Arg Val Pro Val Ser Gln Thr Pro Ala
                   85                  90                  95
   Ile Asn Val Ser Val Pro Thr Thr Leu Pro Ser Ala Thr Gln Val Pro
               100                 105                 110
   Met Glu Val Leu Lys Val Gln Thr His Leu Glu Asn Pro Thr Lys Tyr
           115                 120                 125
   His Ile Gln Gln Ala Gln Arg Gln Gln Val Lys Gln Tyr Leu Ser Thr
       130                 135                 140
   Thr Leu Ala Asn Lys His Ala Asn Gln Val Leu Ser Leu Pro Cys Pro
   145                 150                 155                 160
   Asn Gln Pro Gly Asp His Val Met Pro Pro Val Pro Gly Ser Ser Ala
                   165                 170                 175
   Pro Asn Ser Pro Met Ala Met Leu Thr Leu Asn Ser Asn Cys Glu Lys
               180                 185                 190
   Glu Gly Phe Tyr Lys Phe Glu Glu Gln Asn Arg Ala Glu Ser Glu Cys
           195                 200                 205
   Pro Gly Met Asn Thr His Ser Arg Ala Ser Cys Met Gln Met Asp Asp
       210                 215                 220
   Val Ile Asp Asp Ile Ile Ser Leu Glu Ser Ser Tyr Asn Glu Glu Ile
   225                 230                 235                 240
   Leu Gly Leu Met Asp Pro Ala Leu Gln Met Ala Asn Thr Leu Pro Val
                   245                 250                 255
   Ser Gly Asn Leu Ile Asp Leu Tyr Gly Asn Gln Gly Leu Pro Pro Pro
               260                 265                 270
   Gly Leu Thr Ile Ser Asn Ser Cys Pro Ala Asn Leu Pro Asn Ile Xaa
           275                 280                 285
   Arg Xaa Leu Thr Glu Ser Glu Ala Arg Ala Leu Ala Lys Glu Arg Gln
       290                 295                 300
   Lys Lys Asp Asn His Asn Leu Ile Glu Arg Arg Arg Arg Phe Asn Ile
   305                 310                 315                 320
   Asn Asp Arg Ile Lys Glu Leu Gly Thr Leu Ile Pro Lys Ser Asn Asp
                   325                 330                 335
   Pro Asp Met Arg Trp Asn Lys Gly Thr Ile Leu Lys Ala Ser Val Asp
               340                 345                 350
   Tyr Ile Arg Lys Leu Gln Arg Glu Gln Gln Arg Ala Lys Glu Leu Glu
           355                 360                 365
   Asn Arg Gln Lys Lys Leu Glu His Ala Asn Arg His Leu Leu Leu Arg
       370                 375                 380
   Ile Gln Glu Leu Glu Met Gln Ala Arg Ala His Gly Leu Ser Leu Ile
   385                 390                 395                 400
   Pro Ser Thr Gly Leu Cys Ser Pro Asp Leu Val Asn Arg Ile Ile Xaa
                   405                 410                 415
   Gln Xaa Pro Val Leu Glu Asn Cys Ser Gln Asp Leu Leu Gln His His
               420                 425                 430
   Ala Asp Leu Thr Cys Thr Thr Thr Leu Asp Leu Thr Asp Gly Thr Ile
           435                 440                 445
   Thr Phe Asn Asn Asn Leu Gly Thr Gly Thr Glu Ala Asn Gln Ala Tyr
       450                 455                 460
   Ser Val Pro Thr Lys Met Gly Ser Lys Leu Glu Asp Ile Leu Met Asp
   465                 470                 475                 480
   Asp Thr Leu Ser Pro Val Gly Val Thr Asp Pro Leu Leu Ser Ser Val
                   485                 490                 495
   Ser Pro Gly Ala Ser Lys Thr Ser Ser Arg Arg Ser Ser Met Ser Met
               500                 505                 510
   Glu Glu Thr Glu His Thr Cys
           515
<210> 38
   <211> 419
   <212> PRT
   <213> Homo sapiens
<220>
   <221> SITE
   <222> (182)..(182)
   <223> Xaa = any amio acid
<220>
   <221> SITE
   <222> (184)..(184)
   <223> Xaa = any amio acid
<220>
   <221> SITE
   <222> (316)..(316)
   <223> Xaa = any amio acid
<220>
   <221> SITE
   <222> (318)..(318)
   <223> Xaa = any amio acid
<400> 38
   Met Leu Glu Met Leu Glu Tyr Asn His Tyr Gln Val Gln Thr His Leu
   1               5                   10                  15
   Glu Asn Pro Thr Lys Tyr His Ile Gln Gln Ala Gln Arg Gln Gln Val
               20                  25                  30
   Lys Gln Tyr Leu Ser Thr Thr Leu Ala Asn Lys His Ala Asn Gln Val
           35                  40                  45
   Leu Ser Leu Pro Cys Pro Asn Gln Pro Gly Asp His Val Met Pro Pro
       50                  55                  60
   Val Pro Gly Ser Ser Ala Pro Asn Ser Pro Met Ala Met Leu Thr Leu
   65                  70                  75                  80
   Asn Ser Asn Cys Glu Lys Glu Gly Phe Tyr Lys Phe Glu Glu Gln Asn
                   85                  90                  95
   Arg Ala Glu Ser Glu Cys Pro Gly Met Asn Thr His Ser Arg Ala Ser
               100                 105                 110
   Cys Met Gln Met Asp Asp Val Ile Asp Asp Ile Ile Ser Leu Glu Ser
           115                 120                 125
   Ser Tyr Asn Glu Glu Ile Leu Gly Leu Met Asp Pro Ala Leu Gln Met
       130                 135                 140
   Ala Asn Thr Leu Pro Val Ser Gly Asn Leu Ile Asp Leu Tyr Gly Asn
   145                 150                 155                 160
   Gln Gly Leu Pro Pro Pro Gly Leu Thr Ile Ser Asn Ser Cys Pro Ala
                   165                 170                 175
   Asn Leu Pro Asn Ile Xaa Arg Xaa Leu Thr Ala Cys Ile Phe Pro Thr
               180                 185                 190
   Glu Ser Glu Ala Arg Ala Leu Ala Lys Glu Arg Gln Lys Lys Asp Asn
           195                 200                 205
   His Asn Leu Ile Glu Arg Arg Arg Arg Phe Asn Ile Asn Asp Arg Ile
       210                 215                 220
   Lys Glu Leu Gly Thr Leu Ile Pro Lys Ser Asn Asp Pro Asp Met Arg
   225                 230                 235                 240
   Trp Asn Lys Gly Thr Ile Leu Lys Ala Ser Val Asp Tyr Ile Arg Lys
                   245                 250                 255
   Leu Gln Arg Glu Gln Gln Arg Ala Lys Glu Leu Glu Asn Arg Gln Lys
               260                 265                 270
   Lys Leu Glu His Ala Asn Arg His Leu Leu Leu Arg Ile Gln Glu Leu
           275                 280                 285
   Glu Met Gln Ala Arg Ala His Gly Leu Ser Leu Ile Pro Ser Thr Gly
       290                 295                 300
   Leu Cys Ser Pro Asp Leu Val Asn Arg Ile Ile Xaa Gln Xaa Pro Val
   305                 310                 315                 320
   Leu Glu Asn Cys Ser Gln Asp Leu Leu Gln His His Ala Asp Leu Thr
                   325                 330                 335
   Cys Thr Thr Thr Leu Asp Leu Thr Asp Gly Thr Ile Thr Phe Asn Asn
               340                 345                 350
   Asn Leu Gly Thr Gly Thr Glu Ala Asn Gln Ala Tyr Ser Val Pro Thr
           355                 360                 365
   Lys Met Gly Ser Lys Leu Glu Asp Ile Leu Met Asp Asp Thr Leu Ser
       370                 375                 380
   Pro Val Gly Val Thr Asp Pro Leu Leu Ser Ser Val Ser Pro Gly Ala
   385                 390                 395                 400
   Ser Lys Thr Ser Ser Arg Arg Ser Ser Met Ser Met Glu Glu Thr Glu
                   405                 410                 415
   His Thr Cys
<210> 39
   <211> 413
   <212> PRT
   <213> Homo sapiens
<220>
   <221> SITE
   <222> (182)..(182)
   <223> Xaa = any amino acid
<220>
   <221> SITE
   <222> (184)..(184)
   <223> Xaa = any amino acid
<220>
   <221> SITE
   <222> (310)..(310)
   <223> Xaa = any amino acid
<220>
   <221> SITE
   <222> (312)..(312)
   <223> Xaa = any amino acid
<400> 39
   Met Leu Glu Met Leu Glu Tyr Asn His Tyr Gln Val Gln Thr His Leu
   1               5                   10                  15
   Glu Asn Pro Thr Lys Tyr His Ile Gln Gln Ala Gln Arg Gln Gln Val
               20                  25                  30
   Lys Gln Tyr Leu Ser Thr Thr Leu Ala Asn Lys His Ala Asn Gln Val
           35                  40                  45
   Leu Ser Leu Pro Cys Pro Asn Gln Pro Gly Asp His Val Met Pro Pro
       50                  55                  60
   Val Pro Gly Ser Ser Ala Pro Asn Ser Pro Met Ala Met Leu Thr Leu
   65                  70                  75                  80
   Asn Ser Asn Cys Glu Lys Glu Gly Phe Tyr Lys Phe Glu Glu Gln Asn
                   85                  90                  95
   Arg Ala Glu Ser Glu Cys Pro Gly Met Asn Thr His Ser Arg Ala Ser
               100                 105                 110
   Cys Met Gln Met Asp Asp Val Ile Asp Asp Ile Ile Ser Leu Glu Ser
           115                 120                 125
   Ser Tyr Asn Glu Glu Ile Leu Gly Leu Met Asp Pro Ala Leu Gln Met
       130                 135                 140
   Ala Asn Thr Leu Pro Val Ser Gly Asn Leu Ile Asp Leu Tyr Gly Asn
   145                 150                 155                 160
   Gln Gly Leu Pro Pro Pro Gly Leu Thr Ile Ser Asn Ser Cys Pro Ala
                   165                 170                 175
   Asn Leu Pro Asn Ile Xaa Arg Xaa Leu Thr Glu Ser Glu Ala Arg Ala
   180 185 190
   Leu Ala Lys Glu Arg Gln Lys Lys Asp Asn His Asn Leu Ile Glu Arg
   195 200 205
   Arg Arg Arg Phe Asn Ile Asn Asp Arg Ile Lys Glu Leu Gly Thr Leu
   210 215 220
   Ile Pro Lys Ser Asn Asp Pro Asp Met Arg Trp Asn Lys Gly Thr Ile
   225 230 235 240
   Leu Lys Ala Ser Val Asp Tyr Ile Arg Lys Leu Gln Arg Glu Gln Gln
   245 250 255
   Arg Ala Lys Glu Leu Glu Asn Arg Gln Lys Lys Leu Glu His Ala Asn
   260 265 270
   Arg His Leu Leu Leu Arg Ile Gln Glu Leu Glu Met Gln Ala Arg Ala
   275 280 285
   His Gly Leu Ser Leu Ile Pro Ser Thr Gly Leu Cys Ser Pro Asp Leu
   290 295 300
   Val Asn Arg Ile Ile Xaa Gln Xaa Pro Val Leu Glu Asn Cys Ser Gln
   305 310 315 320
   Asp Leu Leu Gln His His Ala Asp Leu Thr Cys Thr Thr Thr Leu Asp
   325 330 335
   Leu Thr Asp Gly Thr Ile Thr Phe Asn Asn Asn Leu Gly Thr Gly Thr
   340 345 350
   Glu Ala Asn Gln Ala Tyr Ser Val Pro Thr Lys Met Gly Ser Lys Leu
   355 360 365
   Glu Asp Ile Leu Met Asp Asp Thr Leu Ser Pro Val Gly Val Thr Asp
   370 375 380
   Pro Leu Leu Ser Ser Val Ser Pro Gly Ala Ser Lys Thr Ser Ser Arg
   385 390 395 400
   Arg Ser Ser Met Ser Met Glu Glu Thr Glu His Thr Cys
   405 410
<210> 40
   <211> 357
   <212> PRT
   <213> Homo sapiens
<220>
   <221> SITE
   <222> (126)..(126)
   <223> Xaa = any amino acid
<220>
   <221> SITE
   <222> (128)..(128)
   <223> Xaa = any amino acid
<220>
   <221> SITE
   <222> (254)..(254)
   <223> Xaa = any amino acid
<220>
   <221> SITE
   <222> (256)..(256)
   <223> Xaa = any amino acid
<400> 40
   Met Leu Glu Met Leu Glu Tyr Asn His Tyr Gln Val Gln Thr His Leu
   1               5                   10                  15
   Glu Asn Pro Thr Lys Tyr His Ile Gln Gln Ala Gln Arg Gln Gln Gly
               20                  25                  30
   Phe Tyr Lys Phe Glu Glu Gln Asn Arg Ala Glu Ser Glu Cys Pro Gly
           35                  40                  45
   Met Asn Thr His Ser Arg Ala Ser Cys Met Gln Met Asp Asp Val Ile
       50                  55                  60
   Asp Asp Ile Ile Ser Leu Glu Ser Ser Tyr Asn Glu Glu Ile Leu Gly
   65                  70                  75                  80
   Leu Met Asp Pro Ala Leu Gln Met Ala Asn Thr Leu Pro Val Ser Gly
                   85                  90                  95
   Asn Leu Ile Asp Leu Tyr Gly Asn Gln Gly Leu Pro Pro Pro Gly Leu
               100                 105                 110
   Thr Ile Ser Asn Ser Cys Pro Ala Asn Leu Pro Asn Ile Xaa Arg Xaa
           115                 120                 125
   Leu Thr Glu Ser Glu Ala Arg Ala Leu Ala Lys Glu Arg Gln Lys Lys
       130                 135                 140
   Asp Asn His Asn Leu Ile Glu Arg Arg Arg Arg Phe Asn Ile Asn Asp
   145                 150                 155                 160
   Arg Ile Lys Glu Leu Gly Thr Leu Ile Pro Lys Ser Asn Asp Pro Asp
                   165                 170                 175
   Met Arg Trp Asn Lys Gly Thr Ile Leu Lys Ala Ser Val Asp Tyr Ile
               180                 185                 190
   Arg Lys Leu Gln Arg Glu Gln Gln Arg Ala Lys Glu Leu Glu Asn Arg
           195                 200                 205
   Gln Lys Lys Leu Glu His Ala Asn Arg His Leu Leu Leu Arg Ile Gln
       210                 215                 220
   Glu Leu Glu Met Gln Ala Arg Ala His Gly Leu Ser Leu Ile Pro Ser
   225                 230                 235                 240
   Thr Gly Leu Cys Ser Pro Asp Leu Val Asn Arg Ile Ile Xaa Gln Xaa
                   245                 250                 255
   Pro Val Leu Glu Asn Cys Ser Gln Asp Leu Leu Gln His His Ala Asp
               260                 265                 270
   Leu Thr Cys Thr Thr Thr Leu Asp Leu Thr Asp Gly Thr Ile Thr Phe
           275                 280                 285
   Asn Asn Asn Leu Gly Thr Gly Thr Glu Ala Asn Gln Ala Tyr Ser Val
       290                 295                 300
   Pro Thr Lys Met Gly Ser Lys Leu Glu Asp Ile Leu Met Asp Asp Thr
   305                 310                 315                 320
   Leu Ser Pro Val Gly Val Thr Asp Pro Leu Leu Ser Ser Val Ser Pro
                   325                 330                 335
   Gly Ala Ser Lys Thr Ser Ser Arg Arg Ser Ser Met Ser Met Glu Glu
               340                 345                 350
   Thr Glu His Thr Cys
           355

## Description

La présente demande se rapporte au domaine médical, et plus particulièrement à celui de la détermination d'une prédisposition à développer un cancer.

Dans environ 5% des cancers, des mutations constitutionnelle activant des oncogènes ou inactivant des gènes suppresseurs de tumeurs (dits gènes majeurs ou à effet fort), confèrent aux porteurs un risque élevé (supérieur à 50% au cours de la vie) de développer un cancer, à l'origine de formes familiales, du sujet jeune ou d'atteintes multiples. L'expression de la maladie peut varier sous l'influence d'autres facteurs génétiques (dits modificateurs, gènes à effets faibles) ou environnementaux. L'identification des personnes à risque permet de leur faire bénéficier de mesures de prévention et de surveillance visant un dépistage précoce. Dans certains cas, la mutation germinale constitutionnelle peut orienter la thérapie anticancéreuse, c'est le cas des traitements anti-PARP chez les patientes porteuses de mutations germinales de BRCA1 et BRCA2 (Hennessy B.T.J., JCO, 2010, 28, 3570- 3576).

Le mélanome est une tumeur maligne des mélanocytes. C'est un des cancers de la peau les plus rares mais il est cause de la majorité des décès dus au cancer de la peau. Malgré de nombreuses années de recherche intensive, le seul traitement efficace est la résection chirurgicale de la tumeur primaire avant qu'elle n'atteigne une épaisseur de plus d'1 mm. Selon un rapport de l'OMS, il y a environ 48 000 décès liés aux mélanomes par an. Dans plusieurs études, une augmentation du risque de développer un cancer du sein, un lymphome ou un cancer du rein chez les patients ayant des mélanomes cutanés a été discutée.

Concernant le mélanome, deux gènes à effet fort ont été identifiés à ce jour, *CDKN2A* codant les protéines p16^{INK4A} et p14^{ARF}, et *CDK4.* Le facteur environnemental majeur est l'exposition aux UV. Les gènes à effets faibles connus sont principalement des gènes codants des protéines impliquées dans la pigmentation, *MC1R* étant le plus étudié à ce jour. 50% des familles à trois cas de mélanome n'ont pas de gène de prédisposition identifié.

Dans ce contexte, les inventeurs ont étudié le gène *MITF,* gène majeur de régulation du mélanocyte (1) et oncogène (2,3) comme gène candidat de prédisposition au mélanome. MITF est un facteur de transcription de la famille bHLH-LZ qui joue un rôle majeur dans la survie et le développement des mélanocytes. MITF est impliqué dans la régulation de la mélanogenèse. Le rôle de MITF est inhabituel puisqu'il est à la fois inducteur et répresseur de la prolifération cellulaire. En effet, ce facteur est nécessaire d'une part pour la différentiation terminale des mélanocytes et/ou la pigmentation, et d'autre part pour le comportement malin en induisant la prolifération cellulaire. Des mutations constitutionnelle « perte de fonction » de MITF ont été associées à des maladies autosomales dominantes, notamment le Syndrome de Waardenburg et le Syndrome de Tietz, se caractérisant par une surdité avec des anomalies de la pigmentation de la peau, des cheveux et/ou de l'iris.

Le gène MITF comprend 9 exons. Six isoformes de *MITF* ont été identifiées. Chez l'Homme, on les définit généralement comme les isoformes 1 à 6, l'isoforme 4 étant plus connue sous le nom d'isoforme M. Chez la souris, on utilise plutôt la nomenclature avec des lettres. Ces isoformes sont transcrites par des promoteurs spécifiques. De plus, elles se distinguent par leur région N-terminale et contiennent toutes les exons 2 à 9, l'exon 1 étant spécifique de chaque isoforme (1). L'isoforme 4, plus communément appelé MITF-M, se différencie des autres isoformes par une insertion de six acides aminés. Cette isoforme a été détectée uniquement dans des mélanocytes ou des cellules transformées *in vivo* (nevus, mélanome, etc) ou dérivées *in vitro* (lignées). Les autres isoformes sont exprimées dans beaucoup de tissus et de lignées cellulaires, parfois aussi avec des spécificités tissulaires.

WO 00/47765 enseigne que l'épissage alternative du gène MITF produit des transcrits mitf+ et mitf- codant pour des protéines différant par l'insertion de six acides aminés supplémentaires dans mitf+. L'expression de mitf+ et mitf- prédomine respectivement dans les cellules saines et tumorales. Cette demande de brevet divulgue donc une méthode semi-quantitative pour évaluer, prédire, ou surveiller le risque et le traitement de mélanomes. WO 05/116249 décrit également une méthode quantitative basée sur ces variants d'épissage de MITF.

Cronin et al (Pigment Cell & Melanoma Research, 2009, 22, 435-444) et Yokoyama et al (Pigment Cell & Melanoma Research, 2009, 22, 376-377) décrivent des mutations du gène MITF identifiées dans des échantillons de mélanome. Murakami et al (Pigment Cell Research, 2005, 18, 265-277) décrit l'effet de la sumoylation sur l'activité transcriptionnelle de MITF. Miller et al (J. Biol. Chem., 2005, 280, 146-155) décrit des mutations sur les sites de sumoylation de MITF qui abolissent la sumoylation et modifient l'activité transcriptionnelle de MITF.

Dans la présente invention, les inventeurs ont identifié une mutation constitutionnelle récurrente du gène *MITF,* appelée ici E318K (selon la nomenclature de l'isoforme 4), qui est utile comme marqueur de prédisposition au cancer.

La portée de la présente invention est définie par les revendications. Les éléments en dehors de cette portée sont fournis pour information seulement.

Le gène *MITF* (facteur de transcription associé à la microphtalmie) est bien connu de l'homme du métier et peut être caractérisé par son référencement dans des bases de données comme UniGene (Hs.166017), HomoloGene (4892) and GeneID (4286). Il est également appelé MI, WS2A ou bHLHe32.

Cette mutation E318K est localisée dans l'exon 9 du gène *MITF,* en particulier en position 952 de l'isoforme M selon la nomenclature HGVS. Elle consiste en la substitution d'un nucléotide G par un nucléotide A (c.952G>A) et résulte en la substitution d'un acide glutamique par une lysine (p.Glu318Lys). Cet exon 9 est commun à tous les variants d'épissage de MITF. Dans l'isoforme MITF-M, ce résidu est en position 952 du transcrit et provoque la mutation de l'acide aminé en position 318. Le terme « mutation E318K » désigne la mutation, quelque soit sa position dans les isoformes de MITF. Notamment, la position du nucléotide muté et de l'acide aminé dans les différents variants d'épissage est indiquée ci-dessous.

| Isoforme | Autre nom | ref transcrit | Ref protéine | Mutation | SEQ ID No |
|---|---|---|---|---|---|
| ISOFORM 1 | MITF-A | NM_198159 | NP_937802 | E419K | 35 |
| ISOFORM 2 | MITF-H | NM_198177 | NP_937820 | E403K | 36 |
| ISOFORM 3 | MITF-C | NM_006722 | NP_006713 | E418K | 37 |
| **ISOFORM 4** | **MITF-M** | **NM_000248** | **NP_000239** | **E318K** | **38** |
| ISOFORM 5 | | NM_198158 | NP_937801 | E312K | 39 |
| ISOFORM 6 | | NM_198178 | NP_937821 | E394K | 40 |

Le mutant MITF E318K a montré une activité plus forte que MITF sauvage pour l'activation de la transcription du gène HIF1A, ce gène étant connu comme jouant un rôle majeur dans la carcinogenèse rénale (activé secondairement aux mutations « pertes de fonction » des gènes de prédisposition au cancer du rein, tels que VHL, FH, SDHB). De plus, il a été établi que cette mutation diminue la sumoylation de MITF, influençant ainsi peut-être la stabilité de la protéine ou la quantité de protéines codées par les gènes cibles. En effet, l'acide aminé E318 fait partie d'un des deux sites de sumoylation de la protéine MITF. Le gène *MITF* codant un facteur de transcription, la protéine MITF mutée E318K est susceptible d'activer en continu certains de ses gènes cibles. Il est aussi possible que la mutation change la localisation de la protéine MITF ou son ratio nucléo-cytoplasmique.

Les inventeurs ont découvert que le mutant MITF E318K est plus représenté chez des patients présentant un mélanome malin cutané conjointement à un cancer du rein. La mutation serait également plus représentée chez les sujets ayant développé un mélanome cutané malin et un cancer du rein ou dans les familles présentant des mélanomes et des cancers du rein chez des apparentés, ou un mélanome cutané et un autre cancer, notamment une polyglobulie ou un lymphome. Ce mutant est présent à une fréquence très faible (2/2846 sujets, soit une fréquence d'hétérozygotes de 0,0007 dans une première cohorte, et 11/1824 sujets, soit une fréquence d'hétérozygotes de 0,003 dans une deuxième cohorte) chez les sujets sains témoins. Les inventeurs ont montré que la forme non-sumoylée de *MITF* conduit à des mélanocytes moins différentiés et des cellules plus prolifératives.

Par ailleurs, il est probable que ce mutant soit également plus représenté chez les sujets ayant développé une ou des tumeurs issues de la crête neurale telles que le cancer neuroendocrinien, un sarcome, un neuroblastome ou une tumeur du système nerveux (TSN), ou d'autres types de cancers selon les résultats préliminaires des inventeurs tels qu'un lymphome, un cancer du poumon, un cancer du rein, un cancer du sein, un cancer du pancréas, une tumeur pédiatrique, un cancer du système hématopoïétique, un cancer du système digestif, une polyglobulie ou une combinaison de ces types de cancer. Les sites de sumoylation comprennent classiquement le motif consensus W-K-X-E dans lequel W est un acide aminé hydrophobe et X est un quelconque acide aminé. MITF possède deux sites de sumoylation : le premier site ayant la séquence IKRE (avec le K en positions respectives dans les isoformes 1, 2, 3, 4, 5 et 6 : 289, 273, 288, 182, 182 et 126) et le deuxième site ayant la séquence IKQE (avec le K en positions respectives dans les isoformes 1, 2, 3, 4, 5 et 6 : 417, 401, 416, 316, 310 et 254). Les mutations de K182 et K316 augmentent la transcription d'un gène cible, mélastatine/TRPM, mais n'influencent pas la capacité de la protéine à se lier à l'ADN, ni la localisation ou la stabilité de la protéine (Miller AJ et al, JBC, 2005, 280 : 146-155).

L'enseignement pour le mutant E318K de MITF de la présente invention est généralisable à toute mutation de MITF diminuant ou abolissant la sumoylation de la protéine MITF à un des sites de sumoylation ou au deux.

Par conséquent, la présente description est relative à une méthode pour déterminer si un sujet a une prédisposition à ou une susceptibilité de développer un cancer choisi parmi la liste suivante : un mélanome cutané malin, un cancer du rein, un cancer de la thyroïde, un sarcome, un neuroblastome, une tumeur du système nerveux central (TSNC), un lymphome, un cancer du poumon, une polyglobulie, et des combinaisons de ceux-ci, comprenant la détermination dans un échantillon biologique du sujet de la présence d'une mutation dans MITF (facteur de transcription associé à la microphtalmie) diminuant ou abolissant la sumoylation de MITF, la présence de cette mutation indiquant que le sujet a une prédisposition à ou une susceptibilité de développer un tel cancer. Dans un aspect particulier, notamment lorsque la mutation dans MITF est E318K (c'est-à-dire E318K dans l'isoforme 4 ou la substitution du résidu Glu correspondant dans les autres isoformes de MITF par un résidu Lys), le cancer est un mélanome cutané malin ou une combinaison d'un mélanome cutané malin et d'un autre cancer, en particulier un cancer choisi parmi un cancer neuroendocrinien, un sarcome, un neuroblastome ou une tumeur du système nerveux (TSN), un lymphome, un cancer du poumon, un cancer du rein, un cancer du sein, un cancer du pancréas, une tumeur pédiatrique, un cancer du système hématopoïétique, un cancer du système digestif, une polyglobulie et des combinaisons de ceux-ci. Le cancer peut également être choisi parmi un cancer du rein, un cancer de la thyroïde, un sarcome, un neuroblastome, une tumeur du système nerveux central (TSNC), un lymphome, un cancer du poumon, une polyglobulie, et de combinaisons de ceux-ci. Dans un mode de réalisation particulier de l'invention, la combinaison est celle d'un mélanome cutané malin et d'un cancer du rein.

De préférence, la mutation est une substitution d'un résidu lysine et/ou d'un résidu acide glutamique d'un des sites de sumoylation de MITF ou des deux sites. Ainsi, la mutation est la substitution d'un résidu sélectionné dans le tableau ci-dessous par un quelconque des 19 autres acides aminés.

| Isoforme | Ref transcrit | Ref protéine | 1^{er} site de sumoylation | | 2^{ème} site de sumoylation | |
|---|---|---|---|---|---|---|
| ISOFORM 1 | NM_198159 | NP_937802 | K289 | E291 | K417 | E419 |
| ISOFORM 2 | NM_198177 | NP_937820 | K273 | E275 | K401 | E403 |
| ISOFORM 3 | NM_006722 | NP_006713 | K288 | E290 | K416 | E418 |
| **ISOFORM 4** | **NM_000248** | **NP_000239** | **K182** | **E184** | **K316** | **E318** |
| ISOFORM 5 | NM_198158 | NP_937801 | K182 | E184 | K310 | E312 |
| ISOFORM 6 | NM_198178 | NP_937821 | K126 | E128 | K254 | E256 |

Plus précisément, un résidu lysine peut être substitué par un quelconque des 19 autres acides aminés ; et/ou, un résidu acide glutamique peut être substitué par un quelconque des 19 autres acides aminés.

De préférence, la méthode comprend la détection d'une substitution du résidu « K316 » (c'est-à-dire K316 dans l'isoforme 4 ou le résidu Lys correspondant dans les autres isoformes de MITF) et/ou du résidu « E318 » (c'est-à-dire E318 dans l'isoforme 4 ou le résidu Glu correspondant dans les autres isoformes de MITF) par un quelconque des 19 autres acides aminés.

Dans un aspect préféré, la méthode comprend la détection d'une substitution du résidu «E318 » (c'est-à-dire E318 dans l'isoforme 4 ou le résidu Glu correspondant dans les autres isoformes de MITF) par un quelconque des 19 autres acides aminés. Dans un aspect encore plus préféré, la méthode comprend la détection de la mutation « E318K » (c'est-à-dire E318K dans l'isoforme 4 ou la substitution du résidu Glu par un résidu Lys correspondante dans les autres isoformes de MITF).

La mutation peut être détectée au niveau protéique ou nucléique. Les techniques pour identifier la mutation telle que définie ci-dessus dans le gène *MITF* ou ses transcrits (ARNm) sont bien connues de l'homme du métier et incluent notamment et de manière non-exhaustive le séquençage, l'hybridation sélective et/ou l'amplification sélective. Au niveau nucléique, la détection peut se faire sur un échantillon d'ADN génomique, d'ARNm, ou d'ADNc.

Notamment, le séquençage de MITF peut être complet ou partiel. En effet, la méthode peut comprendre uniquement le séquençage de la région comprenant le résidu suspecté d'être muté et même uniquement le séquençage de ce résidu particulier.

Par hybridation sélective est entendu que l'ADN génomique, l'ARN ou l'ADNc est mis en présence d'une sonde spécifique de MITF du mutant et éventuellement d'une sonde témoin spécifique de MITF ne présentant pas cette mutation ou de MITF sauvage. Les sondes peuvent être en suspension ou immobilisées sur un substrat. Typiquement, les sondes seront marquées pour être plus facilement détectables. Les sondes sont notamment des molécules d'acide nucléique simple-brin de 8 à 1000 nucléotides, de préférence de 10 à 800 ou de 15 à 50 nucléotides.

L'acide nucléique peut être amplifié avant détection de la mutation. Ainsi, un couple d'amorces spécifiques des régions entourant la position de la mutation à détecter (c'est-à-dire en amont et en aval) sera conçue. Typiquement, les amorces sont des molécules d'acides nucléiques simple-brin de 5 à 60 nucléotides, de préférence de 8 à 25 nucléotides. Une complémentarité parfaite est préférée puisqu'elle assure une haute spécificité. Cependant, certains mésappariements peuvent être tolérés. Une fois l'amplification du gène *MITF* ou de l'exon contenant la mutation, ou encore d'un de ses transcrits faite, l'amplicon est utilisé pour la détection de la présence de la mutation par séquençage ou hybridation spécifique ou par toute autre technique appropriée connue de l'homme du métier. La mutation peut également être détectée par analyse des courbes de fusion (voir par exemple, WO2007/035806).

La présence de la mutation peut également être détectée par amplification sélective du mutant. Ainsi, un couple d'amorces est préparé, l'une des amorces s'hybridant spécifiquement avec la séquence portant la mutation à détecter. Cette amorce sera capable d'initier l'amplification ou de s'hybrider avec sa cible uniquement si la séquence porte le nucléotide muté. Par conséquent, la présence d'un amplicon indiquerait que MITF porte la mutation testée alors, que l'absence de cet amplicon indiquerait que MITF ne présente pas cette mutation.

Bien entendu, ces techniques peuvent être facilement adaptées par l'homme du métier pour détecter simultanément ou en parallèle plusieurs mutations du ou des site(s) de sumoylation. Ainsi, les techniques permettraient de détecter une ou plusieurs mutations codant pour la substitution de résidus sélectionnés parmi le groupe constitué de K182, E184, K316 et E318 dans l'isoforme 4 de MITF ou des résidus correspondant dans les autres isoformes de MITF.

Lorsque la mutation est détectée au niveau protéique, la méthode utilise un anticorps capable de discriminer entre MITF présentant la mutation à détecter et MITF ne présentant pas cette mutation. Notamment, l'échantillon biologique est mis en contact avec un anticorps spécifique de MITF présentant la mutation à détecter et la présence de complexe immun est détectée. Différentes méthodes permettent de détecter ces complexes immuns comme les méthodes d'ELISA, radio-immuno-essai (RIA) et tests immuno-enzymatiques (IEMA). Par « anticorps » sont également désignés tous les fragments et dérivés d'anticorps conservant la capacité de se fixer spécifiquement au mutant à détecter de MITF par rapport au MITF ne présentant pas cette mutation. Là encore, ces techniques peuvent être facilement adaptées par l'homme du métier pour détecter simultanément ou en parallèle plusieurs mutations du ou des site(s) de sumoylation. Ainsi, les techniques permettraient de détecter une ou plusieurs substitution(s) de résidus sélectionnés parmi le groupe constitué de K182, E184, K316 et E318 dans l'isoforme 4 de MITF ou des résidus correspondant dans les autres isoformes de MITF, par exemple à l'aide d'une combinaison d'anticorps spécifique de chaque mutant de MITF présentant une ou plusieurs de ces substitutions.

De manière alternative, la mutation diminuant ou abolissant la sumoylation de MITF peut être détectée au niveau protéique de manière indirecte. Ainsi, la présence de la mutation peut être détectée en mesurant la sumoylation de MITF, une diminution de la sumoylation par rapport à la protéine MITF sauvage indiquant la présence de la mutation. Les exemples détaillent comment mesurer cette sumoylation. De même, la mutation diminuant la sumoylation, la protéine MITF mutée est stabilisée et donc détectable dans des coupes tissulaires par immunohistochimie ou immunofluorescence. La détection de la mutation ou de la présence/absence de sumoylation pourrait également se faire par spectrométrie de masse (WO/2005/003390).

Dans un aspect particulièrement intéressant de la présente description, la mesure de la sumoylation de *MITF* se fait de manière indirecte par la détermination de la localisation cellulaire de *MITF* détectée par immunohistochimie ou immunofluorescence. En effet, de manière surprenante et très originale, la diminution de la sumoylation de la protéine *MITF* entraîne une modification de la localisation cellulaire de la protéine *MITF* visible par immunohistochimie dans les cellules tumorales. La protéine sauvage présente une localisation uniquement ou essentiellement nucléaire alors que la protéine portant la mutation diminuant la sumoylation de la protéine, en particulier la mutation E318K, présente une localisation à la fois nucléaire et cytoplasmique. Ainsi, la présente description est relative à une méthode dans laquelle la mutation diminuant la sumoylation de *MITF* est détectée par la détermination de la localisation cellulaire de *MITF* en immunohistochimie, une localisation nucléaire étant indicatif de la protéine MITF sauvage alors qu'une localisation nucléaire et cytoplasmique étant indicatif de la protéine *MITF* présentant une mutation diminuant la sumoylation de *MITF,* en particulier *MITF* mutée E318K. La mutation peut aussi permettre une détection par IHC ou IF de la protéine MITF dans des tissus où la forme sauvage est indétectable par ces mêmes méthodes.

La mutation peut être détectée dans une quelconque isoforme de MITF. Dans un aspect particulier, la mutation est détectée dans l'isoforme 4.

Le terme « sujet » réfère ici à un mammifère, de préférence à un humain.

Le terme « échantillon biologique » réfère ici à un échantillon de tissu sain ou tumoral, par exemple une biopsie et notamment une biopsie de peau, de rein, de thyroïde, ou de poumon, ou de liquide biologique, par exemple un échantillon de sang, de liquide céphalo-rachidien, d'urine, ou de lymphe. De préférence, l'échantillon biologique est un échantillon de sang. Les méthodes de la présente description peuvent comprendre une étape préalable de prélèvement de l'échantillon biologique.

Bien entendu, les méthodes de la présente description considèrent également, outre la détection de la mutation diminuant ou abolissant la sumoylation de MITF, la détection d'autres marqueurs de prédisposition au cancer.

La présente description est également relative à l'utilisation de moyens de détection de la mutation diminuant ou abolissant la sumoylation de MITF pour la préparation d'un kit de diagnostic destiné à déterminer si un sujet a une prédisposition à ou une susceptibilité de développer un cancer choisi parmi la liste suivante : un mélanome cutané malin, un cancer neuroendocrinien, un sarcome, un neuroblastome ou une tumeur du système nerveux (TSN), un lymphome, un cancer du poumon, un cancer du rein, un cancer du sein, un cancer du pancréas, une tumeur pédiatrique, un cancer du système hématopoïétique, un cancer du système digestif, une polyglobulie, et des combinaisons de ceux-ci, la présence de cette mutation indiquant que le sujet a une prédisposition à ou une susceptibilité de développer un tel cancer. Dans un aspect particulier, le cancer est choisi parmi un mélanome cutané malin, un cancer du rein, un cancer de la thyroïde, un sarcome, un neuroblastome, une tumeur du système nerveux central (TSNC), un lymphome, un cancer du poumon, un polyglobulie et des combinaisons de ceux-ci. De manière préférée, le cancer est choisi parmi un mélanome cutané malin, un cancer du rein, et une combinaison de ceux-ci. Les moyens de détection peuvent comprendre ou consister en une sonde spécifique de MITF portant la mutation à détecter, un couple d'amorces permettant l'amplification d'un segment nucléotidique comprenant la mutation à détecter, un couple d'amorces dont l'une d'elles s'hybride spécifiquement avec la séquence portant la mutation à détecter (permettant ainsi une amplification sélective du mutant à détecter de MITF), un anticorps spécifique du mutant à détecter de MITF, des moyens permettant la détection et la mesure de la sumoylation de MITF, des contrôles négatifs permettant de détecter MITF ne présentant pas la mutation à détecter, ou des combinaisons de ceux-ci. Dans un aspect particulier, notamment lorsque la mutation dans MITF est E318K (c'est-à-dire E318K dans l'isoforme 4 ou la substitution du résidu Glu correspondant dans les autres isoformes de MITF par un résidu Lys), le cancer est un mélanome cutané malin ou une combinaison d'un mélanome cutané malin et d'un autre cancer, en particulier d'un cancer choisi parmi cancer neuroendocrinien, un sarcome, un neuroblastome ou une tumeur du système nerveux (TSN), un lymphome, un cancer du poumon, un cancer du rein, un cancer du sein, un cancer du pancréas, une tumeur pédiatrique, un cancer du système hématopoïétique, un cancer du système digestif, une polyglobulie, et des combinaisons de ceux-ci. Dans un aspect particulier, le cancer est choisi parmi un mélanome cutané malin, un cancer du rein, un cancer de la thyroïde, un sarcome, un neuroblastome, une tumeur du système nerveux central (TSNC), un lymphome, un cancer du poumon, un polyglobulie et des combinaisons de ceux-ci. De manière préférée, le cancer est choisi parmi un mélanome cutané malin, un cancer du rein, et une combinaison de ceux-ci.

L'intérêt de la détection d'une prédisposition ou d'une susceptibilité au cancer est que le sujet peut bénéficier d'un suivi clinique ou d'une surveillance permettant de détecter l'apparition d'un cancer à un stade précoce et donc d'augmenter les chances de guérison. Par ailleurs, la détection de la mutation peut permettre d'orienter la prise en charge thérapeutique du patient et/ou d'augmenter l'efficacité des traitements. Par ailleurs, le sujet identifié peut également bénéficier d'un traitement préventif. Ce traitement est destiné à empêcher ou retarder l'apparition du cancer.

Ainsi, la présente description est également relative à une méthode pour sélectionner les patients pouvant bénéficier d'un traitement préventif ou d'une surveillance médicale comprenant la détermination de la susceptibilité au cancer du patient par la méthode selon la présente description et la sélection des sujets présentant la mutation diminuant ou abolissant la sumoylation de MITF.

Le traitement préventif envisagé peut comprendre l'administration de composés polyphénoliques. En effet, il a été notamment montré que des composés polyphénoliques de l'enveloppe du riz fermentée diminuent le niveau de protéine MITF (5). Ainsi, ce traitement pourrait contrecarrer la diminution de la sumoylation de la protéine MITF et ses conséquences fonctionnelles sur ses gènes cibles, MITF étant un facteur de transcription. Par ailleurs, les polyphénols du thé noir ont une action chimiopréventive qui pourrait se faire par un arrêt du cycle cellulaire et par un mécanisme pro-aptotique (6). Par conséquent, la présente description est relative aux composés polyphénoliques pour une utilisation dans le traitement préventif du cancer chez des sujets présentant une mutation de MITF diminuant ou abolissant la sumoylation de MITF et l'utilisation de composés polyphénoliques pour la préparation d'un médicament destiné au traitement préventif du cancer (chimioprévention) chez des sujets présentant une mutation de MITF diminuant ou abolissant la sumoylation de MITF. Elle concerne en outre une méthode de traitement comprenant l'administration d'une dose thérapeutique efficace de composés polyphénoliques à des sujets présentant une mutation de MITF diminuant ou abolissant la sumoylation de MITF, prévenant ou retardant ainsi l'apparition d'un cancer. De préférence, les composés polyphénoliques sont des composés polyphénoliques de l'enveloppe du riz fermentée ou des polyphénols du thé noir. La demande de brevet WO 05/099721 décrit également de nombreux autres composés polyphénoliques utiles pour la prévention du cancer, notamment via leur effet antioxydant.

L'invention sera mieux comprise à l'aide des exemples qui suivent, lesquels se veulent illustratifs et non limitatifs.

### BREVE DESCRIPTION DES FIGURES

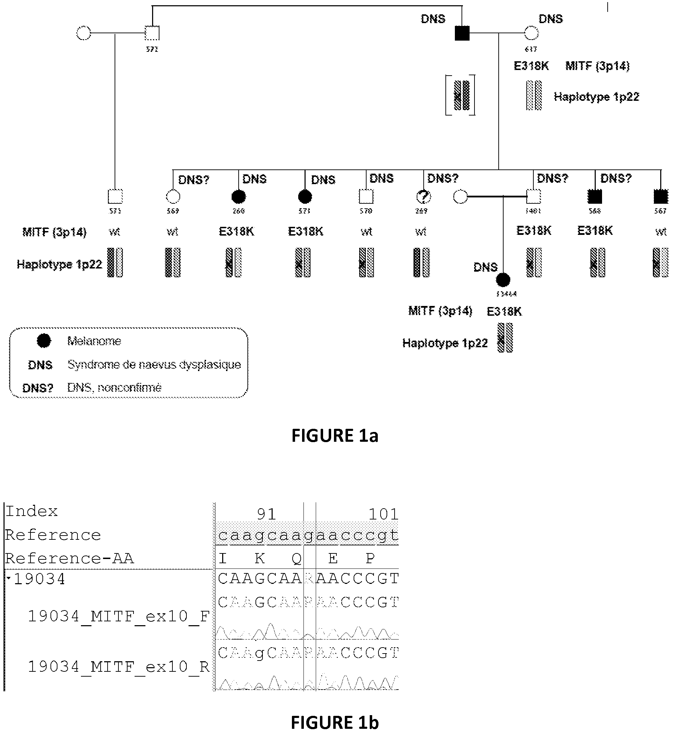

Figure 1 : (Fig 1a) Arbre généalogique de la première famille à cas multiples de mélanomes dans laquelle le mutant MITF E318K a été identifié ; (Fig 1b) électrophorégramme de la mutation constitutionnelle (sang) chez une personne atteinte de mélanome et cancer du rein.

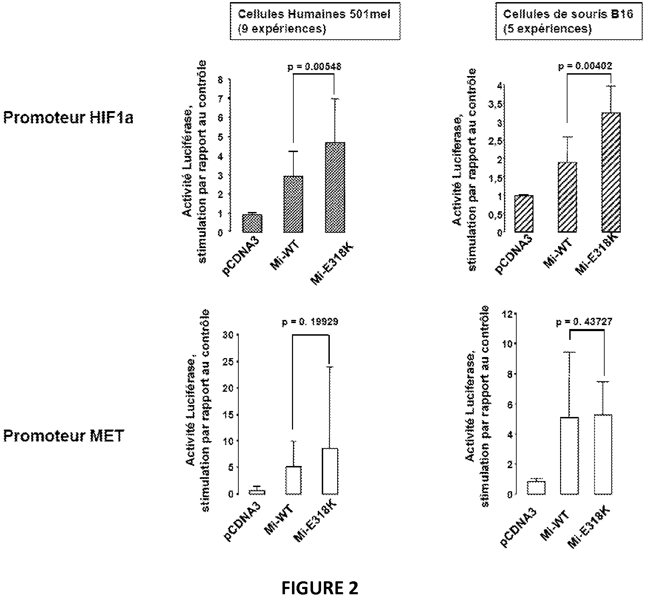

Figure 2 : Activation de la transcription du promoteur de HIF1A mais pas de MET en présence de la protéine MITF portant la mutation E318K par rapport à la protéine MITF sauvage.

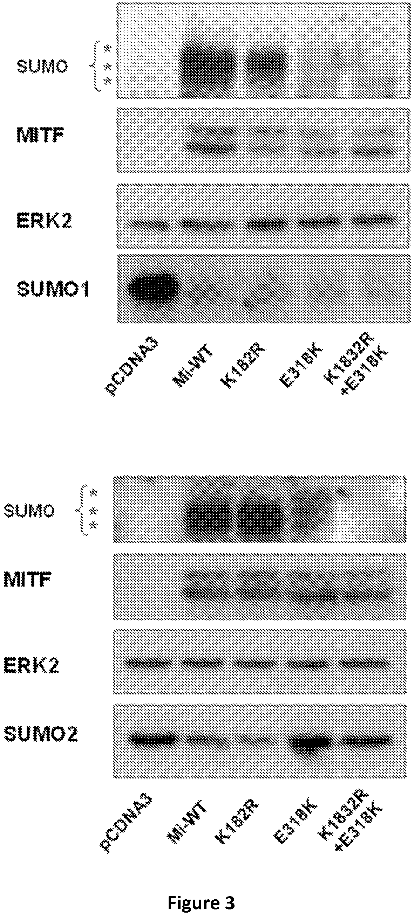

Figure 3 : Diminution de la sumoylation de la protéine MITF portant la mutation E318K.

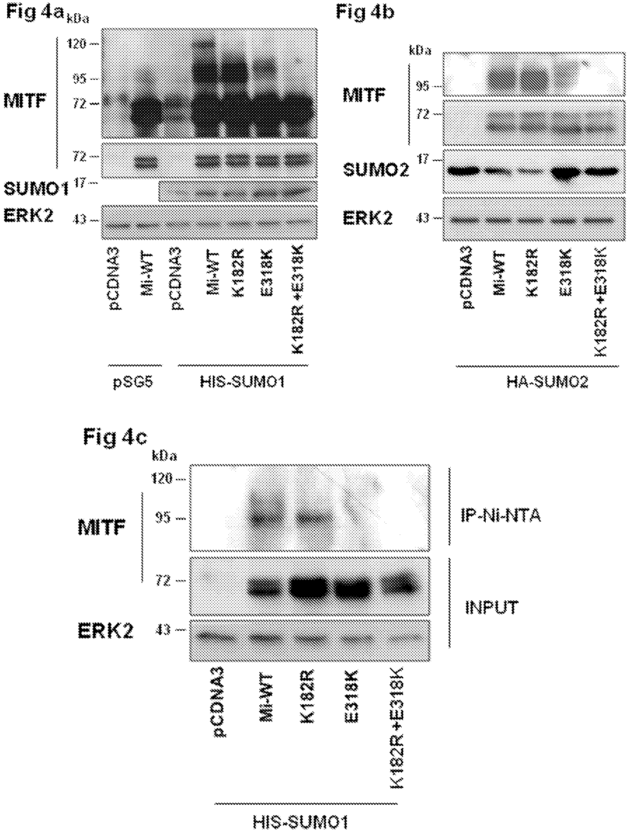

Figure 4 : La mutation E318K diminue la sumoylation de la protéine MITF. Fig 4a) Des cellules HEK293 ont été co-transfectées avec un plasmide codant pour MITF couplée à myc sauvage ou mutée (K182R ; E318K ; K182R/E318K) et un plasmide vide pSG5 ou codant pour His-SUMO1 Les cellules ont été lysées dans un tampon bouillant et testées par western blot pour MITF et ERK2 pour s'assurer la charge suffisante de chaque ligne. Fig 4b) Des cellules HEK293 ont été co-transfectées avec un plasmide codant pour MITF couplée à myc sauvage ou mutée (K182R; E318K; K182R/E318K) et un plasmide vide pSG5 ou codant pour His-SUMO2. Les cellules ont été testées par western blot pour MITF et ERK2. Fig 4c) Des cellules HEK293 ont été co-transfectées avec un plasmide codant pour MITF couplée à myc sauvage ou mutée (K182R; E318K; K182R/E318K) et un plasmide vide pSG5 ou codant pour His-SUMO1. Les lysats cellulaires ont été purifiés en utilisant des colonnes Ni-NTA et analysés par western blot pour MITF (panneau du haut). Un western blot a été fait sur les lysats cellulaires avant purification pour contrôler l'expression de MITF et la charge suffisante de chaque ligne (panneau du bas).

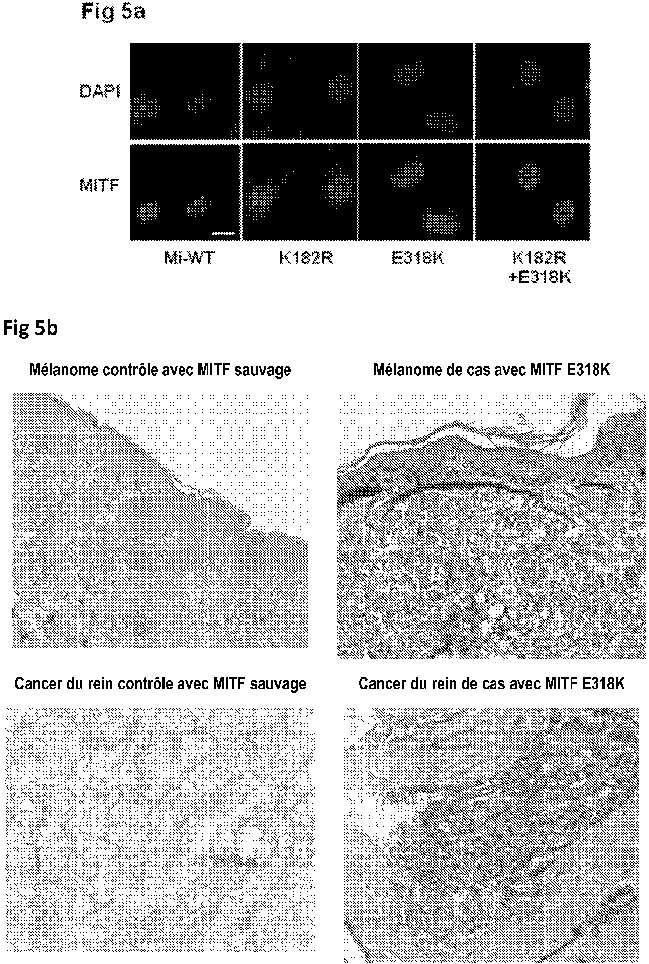

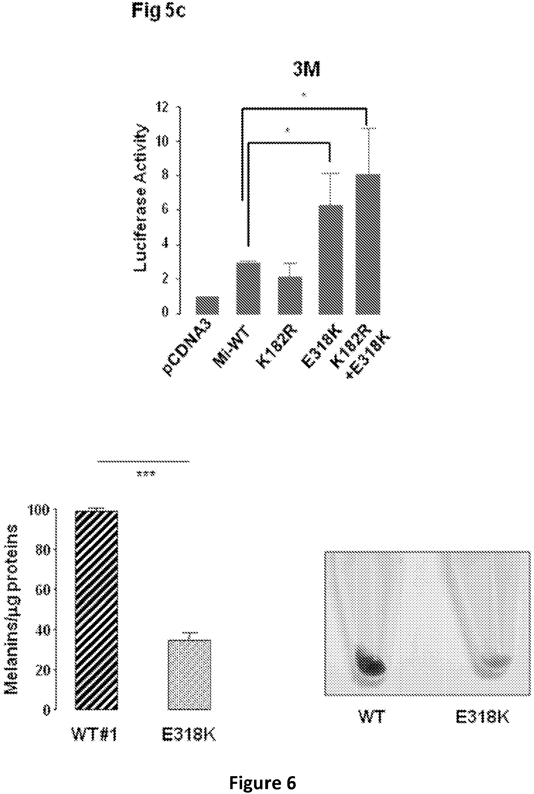

Figure 5 : La mutation E318K pourrait affecter la localisation cellulaire de MITF et changer son activité transcriptionnelle. Fig 5a) Une analyse par immunofluorescence de cellules HEK293 transfectées avec un plasmide codant pour MITF couplée à myc sauvage ou mutée (K182R ; E318K ; K182R/E318K) et colorées avec un anti-myc suivi par un anticorps secondaire anti-souris marqué par Alexafluor 594. Les noyaux cellulaires ont subi une coloration par contraste avec du DAPI. La barre représente 10 µm. Fig 5b) Coloration immunohistochimique de mélanomes et de cancers du rein avec des anticorps anti-MITF (x200). Les mélanomes avec MITF sauvage montrent une coloration nucléaire alors que les mélanomes avec MITF muté E318K montrent une coloration nucléaire et cytosplasmique. Le cancer du rein avec MITF sauvage ne montre pas de marquage alors que le cancer du rein avec MITF muté E318K montrent une coloration nucléaire et cytosplasmique. Fig 5c) Des cellules HEL293 ont été transfectées de manière transitoire avec un plasmide rapporteur luciférase synthétique qui contient 3 copies d'une boite M et avec un plasmide pCDNA3 vide ou codant MITF sauvage ou mutée. L'activité luciférase a été normalisée par l'activité β-galactosidase et les résultats ont été exprimés comme le facteur de stimulation de l'activité luciférase de base pour des cellules non-stimulées.
Figure 6 : Des mélanocytes mutants montrent une pigmentation plus faible. Les culots cellulaires et la mesure de mélanine dans les mélanocytes de deux donneurs sains par rapport à des mélanocytes isolés d'une biopsie de peau de patients présentant la mutation E318K. (***) indique une différence significative avec une p-value inférieure à 0.001 entre les tests. Les deux donneurs ainsi que les porteurs sont d'origine caucasienne.

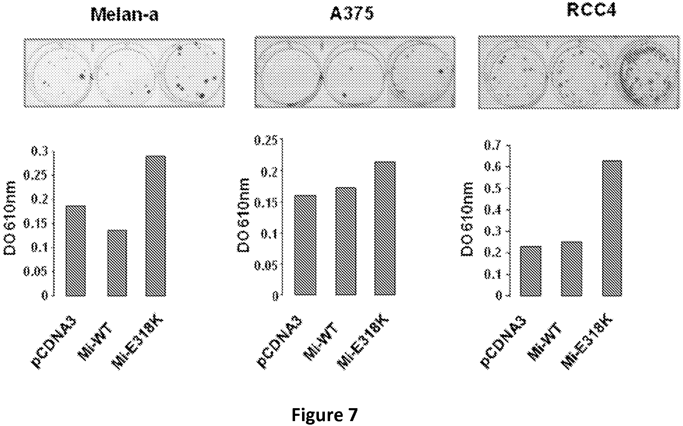

Figure 7 : La mutation E318K confère un avantage de croissance. Des mélanocytes de souris immortalisés melan-a (gauche), des cellules de mélanome humain métastasique A375 (milieu) et des cellules de carcinome rénal humain RCC4 (droite) ont été transfectés avec un vecteur vide, un vecteur codant la protéine MITF sauvage (Mi-WT) ou mutante (Mi-E318K). Des photos à 2 semaines (en haut) et l'absorbance après coloration/décoloration au violet cristal des clones (en bas) sont montrées.

### EXEMPLES

### Exemple 1

Les inventeurs ont étudié le gène MITF, considéré comme un oncogène et donc comme gène candidat. Ils ont d'abord étudié s'il existait des mutations du gène MITF (3p14) dans le site de clivage par les caspases (effet anti-apoptotique) dans des familles à cas multiples de mélanome (4), par séquençage de toutes les isoformes. Leurs résultats préliminaires ont montré la présence d'un variant germinal de MITF-M c.952G>A, p.Glu318Lys (E318K) dans une famille d'origine basque espagnole (TRY) à cas multiples de mélanome (avec 2 cancer du rein, 3 tumeurs du système nerveux central, un cancer du poumon et un cancer gastrique du coté maternel d'où vient la E318K), avec transmission coté paternel d'un haplotype 1p22. Le variant était absent chez 180 témoins français/caucasiens et 96 basques espagnols (Figure 1 a et b).

Dans des cellules de mélanome B16 (souris), les inventeurs ont montré que le variant MITF, c.952G>A, p.Glu318Lys (E318K) avait une action supérieure à la forme sauvage pour induire la transcription du gène HIF1a et pas celle du gène MET (Figure 2). Les inventeurs ont donc posé l'hypothèse suivante : HIF est le facteur transformant des cellules rénales en l'absence de VHL, en situation d'hypoxie; VHL (mutations pertes de fonction d'un gène suppresseur de tumeur) et MET (mutations activatrices d'un oncogène) sont deux gènes de prédisposition au cancer du rein ; HI1aF activé par MITF muté E318K pourrait donc avoir le même effet que l'absence de VHL. Les inventeurs ont donc montré que la mutation E318K du gène MITF était présente chez 4/55 patients et absente chez 276 témoins (p= 0,0007) de la sous-collection « mélanome et cancer du rein (cas sporadiques) » de la collection MELARISK. Les inventeurs ont ensuite exploré la fréquence du mutant E318K dans différentes collections biologiques de l'IGR (Institut Gustave Roussy, Villejuif, France). D'autres porteurs de cette mutation ont été identifiés : une patiente ayant développé un cancer du rein juvénile à translocation TFE3 dont la mère avait eu un cancer du sein puis un mélanome, une patiente ayant développé 3 mélanomes et un lymphome; un cas index d'une famille à cas multiple de mélanome; un cas index ayant développé plusieurs mélanomes ; deux patients ayant développé des mélanomes nodulaires ; une patiente ayant développé un mélanome, un de ses oncles ayant eu une TSNC ; deux patients ayant développé un cancer papillaire du rein ; un patient atteint de polyglobulie. En effet, VHL est l'un des trois gènes de prédisposition à la polyglobulie.

**Tableau 1 : Résultats du criblage du mutant MITF E318K**

| **Mélanome et cancer du rein** | Nb échantillon | MITF sauvage | MITF E318K | p-value |
|---|---|---|---|---|
| Sporadique | 55 | 51 | 4 | 0,0007 |
| Familial | 50 | 49 | 1 | 0,1534 |
| **Sporadique et familial** | **105** | **100** | **5** | **0,0015** |
| **Cancer papillaire du rein** | 24 | 22 | 2 | 0,0062 |
| **Mélanome et TNSC, familial** | | | 1 | 0,1687 |
| **Polyglobulie** | 14 | 13 | 1 | 0,0483 |
| **Lymphome** | 16 | 15 | 1 | 0,0548 |
| **Mélanomes** | | | | |
| Mélanomes multiples Familiaux | 107 | 106 | 1 | 0,2794 |
| Mélanomes multiples Sporadiques | 34 | 33 | 1 | 0,1097 |
| Mélanomes nodulaires | 90 | 88 | 2 | 0,0600 |
| Mélanomes multiples cutanés | 49 | 48 | 1 | 0,1508 |
| familiaux | | | | |

Les populations témoins utilisées sont celles des donneurs du sang et du CEPH (N=276).

### Exemple 2

### Identification de mutations germinales de MITF dans des patients présentant des mélanomes et des cancers du rein

Dans le but de confirmer l'identification du variant de *MITF* résultant d'une substitution faux-sens p.E318K (c.952G>A, dans l'isoforme M de *MITF,* NM_000248) associés à une coexistence de mélanomes et de cancers du rein, les inventeurs ont séquencé la séquence codante entière du gène, les liaisons intron-exon et les 8 promoteurs alternatifs chez 62 patients qui présentent à la fois un mélanome et un cancer du rein. Les inventeurs ont observé cette substitution dans 5 des 62 patients. La fréquence de ce variant est significativement plus élevée que dans une population contrôle de 1824 personnes (4% vs 0,3%, p = 9,7 x 10⁻⁵). Ainsi, porter le variant p.E318K conduit à une augmentation de 14 fois pour le risque de développer à la fois un mélanome et un cancer du rein (risque relatif approché (OR) = 14,46 [intervalle de confiance de 95% = 3,79 à 46,82] (Tableau 2)). Pour confirmer que ce variant a un effet sur la susceptibilité à un mélanome seul, les inventeurs ont génotypé 704 patients présentant des mélanomes (qui sont négatifs pour les mutations de prédisposition aux mélanomes de *CDKN2A* et *CDK4*) incluant 422 cas indépendants avec des antécédents familiaux de mélanomes, 242 cas sporadiques avec des mélanomes multiples primaires et 40 cas sporadiques avec un mélanome nodulaire (Tableau 2). Les derniers cas ont été testés car 4 patients sur 5 présentant développer à la fois un mélanome et un cancer du rein et portant la mutation p.E318K ont un moins un mélanome nodulaire (celui-ci étant le type histologique de mélanome le plus rare). La fréquence de p.E318K est significativement plus élevée chez tous les patients présent un mélanome seul par rapport aux contrôles (1,3% vs 0,3%, p = 4,5 x 10⁻⁵) et le fait de porter la mutation p.E318K est associé à une augmentation du risque de mélanome supérieure à 4 fois (risque relatif approché (OR) = 4,57 [intervalle de confiance de 95% = 2,05 à 10,68]). Cette augmentation du risque semble principalement due au groupe de patients sporadiques à mélanomes primaires multiples (risque relatif approché (OR) = 7,10 [intervalle de confiance de 95% = 2,67 à 18,62]) alors que l'effet de p.E318K n'était pas significatif chez les patients à mélanome avec des antécédents familiaux de mélanomes (risque relatif approché (OR) = 2,78 [intervalle de confiance de 95% = 0,91 à 7,90]) ou chez les patients avec des mélanomes nodulaires (risque relatif approché (OR) = 8,67 [intervalle de confiance de 95% = 0,9 à 41,70] (Tableau 3)). Cependant, le test d'homogénéité de la fréquence de l'allèle p.E318K à travers ces trois groupes étant seulement marginalement significatif (p = 0,06). Le matériel biologique était disponible pour des membres supplémentaires affectés dans trois des sept familles sujettes aux mélanomes dans lesquels le proposant portait le variant. Dans chacune des ces familles, p.E318K coségrège avec le mélanome. De manière alternative, pour examiner l'effet de p.E318K sur la susceptibilité au cancer du rein, les inventeurs ont génotypé ce variant dans 187 patients ayant un cancer du rein. La fréquence de p.E318K était également plus élevée chez les patients ayant un cancer du rein que des les contrôles (1,3% vs 0,3%, p = 0,01) et l'augmentation du risque de cancer du rein associé avec p.E318K était du même ordre de grandeur que celui observé pour le mélanome seul (risque relatif approché (OR) = 4,53 [intervalle de confiance de 95% = 1,22 à 14,30]) (tableau 2). Il n'y avait aucune preuve significative d'une hétérogénéité de p.E318K, la fréquence de l'allèle à travers les trois groupes de patients (mélanome+cancer du rein, mélanome seul et cancer du rein seul ; p = 0,08). Le regroupement de tous les patients augmente la significativité de la différence de fréquence de l'allèle p.E318K entre les cas et les contrôles (1,5% vs 0,3% ; p = 2,5 x 10⁻⁷). Dans l'ensemble, les porteurs de p.E318K ont une augmentation du risque de développer un mélanome, un cancer du rein ou les deux de plus de 5 fois (risque relatif approché (OR) = 5,17 [intervalle de confiance de 95% = 2,49 à 11,52]).

Pour estimer si p.E318K prédispose à l'association de mélanomes et d'un autre cancer différent du cancer du rein, les inventeurs ont génotypé 172 patients affectés par un mélanome et une autre tumeur primaire mais aucun porteur de p.E318K n'a pu être identifié (Tableau2). Puisque l'association de p.E318K avec une autre tumeur primaire est un évènement rare, les inventeurs ont planifié l'examen de séries plus larges.

**Tableau 2 : Fréquence de la mutation germinale de MITF p ;E318K chez des patients souffrant de cancer**

| **Type de tumeur** | **Nombre De Non-porteur** | **Nombre De porteur *** | **Total** | **Fréquence allèle minoritaire** | **FET p-value** | **OR [95 % CI]** |
|---|---|---|---|---|---|---|
| **Contrôles** | **1813** | **11** | **1824** | **0.003** | **-** | **Ref** |
| **CM or/and RCC** | **924** | **29** | **953** | **0.015** | **2.5x 10⁻⁷** | **5.17 [2.49-11.52]** |
| Les deux | 57 | 5^{a} | 62 | 0.040 | 9.7x 10⁻⁵ | 14.46 [3.79-46.82] |
| CM seul^{b} | 685 | 19^{c} | 704 | 0.013 | 4.5x 10⁻⁵ | 4.57 [2.05-10.68] |
| RCC seul^{d} | 182 | 5^{e} | 187 | 0.013 | 0.012 | 4.53 [1.22-14.30] |
| **Melanome et autre cancer^{f}** | **172** | **0** | **172** | **0** | **0.61** | - |

| | | | | | | |
|---|---|---|---|---|---|---|
| OR = risque relatif approché ; 95%CI = intervalle de confiance de 95% ; p-value = probabilité critique du test ; CM = mélanome ; RCC = cancer du rein. * Tous les porteurs sont hétérozygotes pour le variant p.E318K. ^{a} Les 5 patients ont développé un cancer du rein à cellules claires (ccRCC), 4 parmi les 5 patients ont développé au moins un mélanome nodulaire, et le 5^{ème} patient a développé un mélanome à extension superficielle (SSM). ^{b} Mélanomes familiaux (notamment avec au moins 2 cas confirmés de mélanomes dans la famille), 422 cas; cas sporadiques avec des mélanomes primaires multiples (MPM), 242 cas ; mélanome nodulaire sporadique, 40 cas. ^{c} Sur 19 porteurs, 7 sont des cas familiaux, 9 sont des cas sporadiques MPM et 2 sont des cas de melanoma nodulaire sporadique. ^{d} Cancer du rein à cellules claires (ccRCC), 54 cas sporadiques; carcinome papillaire rénal (PRC), 55 cas (22 cas ayant un type I, 30 cas ayant un type II, et 3 cas ayant un sous-type histologique inconnu) ; des phénotypes de cancer du rein mixtes (notamment, papillaire et à cellules claires), 2 cas ; cancer du rein pédiatrique, 5 cas ; Cancer du rein avec un sous-type histologique inconnu, 71 cas. ^{e} Sur 5 porteurs, un est un cas de cancer du rein à cellules claires ccRCC, 2 sont des cas de carcinome papillaire rénal de type II, 1 est un carcinome juvénile avec une translocation somatique t(X;17)(p11;q25) et 1 est un cas de carcinome papillaire rénal de type I. ^{f} Cancer du sein, 97 cas, cancer du cerveau, 27 cas, carcinome de la thyroïde non-médulaire, 28 cas, cancer du colon, 10 cas ; autres cancers (testicule, utérus, ovaire, prostate, sarcome et endomètre), 10 cas. Test d'homogénéité des fréquences allèliques parmi les mélanomes seuls, les cancers du rein seuls et la combinaison des deux : p = 0.078 (test exact) | | | | | | |

**Tableau 3 : Fréquence de la mutation germinale de MITF p ;E318K chez des patients souffrant de mélanome seul**

| **Type de tumeur** | **Nombre De Non-porteur** | **Nombre De porteur *** | **Total** | **Fréquence allèle minoritaire** | **FET p-value** | **OR [95 % CI]** |
|---|---|---|---|---|---|---|
| **Contrôles** | **1813** | **11** | **1824** | **0.003** | **-** | **Ref** |
| **CM seul** | **685** | **19** | **704** | **0.013** | **4.5x 10⁻⁵** | **4.57 [2.05-10.68]** |
| Familial | 415 | 7 | 422 | 0.008 | 0.06 | 2.78 [0.91-7.90] |
| Mélanome primaire multiple (MPM) | 232 | 10 | 242 | 0.021 | 4.1 x 10⁻⁵ | 7.10 [2.67-18.62] |
| Mélanome nodulaire | 38 | 2 | 40 | 0.025 | 0.029 | 8.67 [0.90-41.70] |

| | | | | | | |
|---|---|---|---|---|---|---|
| Test d'homogénéité des fréquences allèliques parmi les 3 catégories de mélanomes seuls (familial, MPM, nodulaire): p = 0.065 (test exact) si le test d'homogénéité est fait en comparant familial versus MPM: p = 0.072 (test exact) | | | | | | |

### Exemple 3

### Conséquences fonctionnelles de la mutation p.E318K de MITF

Cette mutation E318K est située dans l'un des deux sites de sumoylation de la protéine (motif WKXE, 2 sites K182 et K316). Les inventeurs ont montré une diminution de la sumoylation de MITF en présence de la mutation E318K. (Figure 3).

Plus précisément, les inventeurs ont généré par mutagenèse dirigée le variant E318K. Par ailleurs, ils ont également préparé le variant K182R et un double mutant K182R :E318K.

Des expériences de Western blot sur des extraits totaux avec un anticorps anti-*MITF,* après coexpression de His-SUMO-1 avec *MITF* sauvage ont révélé la présence d'une bande à 120 kD et un doublet à environ 90 kD, suggérant que MITF subit une sumoylation qui augment son poids molécuaire (Figure 4A). Bien que l'ajout de proétines SUMO exogène a augmenté le niveau global de sumoylation de *MITF,* le western blot a montré que *MITF* est également sumoylé dans les conditions de base montrant le doublet à 90 kD, excluant ainsi un effet non-spécifique de la sur-expression de SUMO. La mutation K182R a conduit à la disparition complète de la forme de *MITF* de poids moléculaire le plus élevé, mais n'affecte pratiquement pas la bande à 90 kD. Lorsque le codon 318 a été muté par une lysine, les inventeurs ont observé une diminution importante du niveau de toutes les bandes de haut pois moléculaire de *MITF.* Enfin, aucune forme de haut pois moléculaire de *MITF* n'a pu être observée lorsque le double mutant a été utilisé. Des observations similaires ont été faites avec une co-expression de HA-SUMO-2 (Figure 4B) démontrant que le *MITF* sauvage a été modifié par SUMO1 ou SUMO2 et que la mutation E318K affecte à la fois les modifications de SUMO1 et SUMO2. Pour confirmer l'attachement de SUMO-1 à *MITF,* le plasmide His-SUMO-1 a été transfecté seul ou avec les constructions *MITF.* Alors, les protéines contenant His-SUMO-1 ont été purifiées sur une colonne Ni-TFA. Dans les cellules transfectées avec *MITF* sauvage, le western blot avec un anti-*MITF* a révélé des formes de *MITF* sumoylées migrant à environ 90 et 120 kD (Figure 4C). La mutation K182R affecte principalement la forme sumoylée de *MITF* à 120 kD alors qu'aucune forme sumoylée de *MITF* n'a pu être observée avec E318K ou le double-mutant. Dans l'ensemble, ces résultats démontrent que le codon 316 est un site accepteur majeur de SUMO dans *MITF* et que la mutation E318K diminue drastiquement la sumoylation de *MITF.*

### Exemple 4

### La mutation E318K pourrait changer la localisation de MITF et elle altère son activité transcriptionnelle

La sumoylation orchestre de multiples processus cellulaires en partie par le contrôle de la transduction du signal nucléo-cytoplasmique et de la transcription. Les inventeurs ont examiné si le mutant E318K avec une sumoylation diminuée pouvait altérer la localisation cellulaire de *MITF* (Figure 5A). La coloration par immunofluorescence avec un anticorps anti-*MITF* a montré que le mutant E318K mais également K182R et K182R :E318K ont été détectés dans le noyau des cellules de mélanome, ce qui est cohérent avec la localisation nucléaire de la forme sauvage de *MITF.* Cependant, des expériences d'immuno-histochimie ont permis de montrer, avec des échantillons de mélanome et de cancer du rein, un marquage nucléaire et cytoplasmique pour les mutants E318K (Figure 5B).

| Type de marquage | Nucléaire | Nucléaire+cytoplasmique | Aucun |
|---|---|---|---|
| Mélanome contrôle | 8/9 | 1/9 | 0/9 |
| Mélanome porteur de E318K | 0/8 | 8/8 | 0/8 |
| Cancer du rein contrôle | 0/6 | 0/6 | 6/6 |
| Cancer du rein porteur de E318K | 0/6 | 2/6 | 4/6 |

Les inventeurs ont également exploré l'effet possible de la substitution E318K sur l'activité transcriptionnelle de *MITF.* Ils ont comparé l'activité de la forme sauvage versus le mutant E318K sur un rapporteur synthétique qui contient 3 copies d'une boite M apposée à un promoteur minimal SV40. Le mutant E318K a montré une activité transcriptionnelle 2-3 fois plus élevée que celle de la forme sauvage (Figure 5C). Le double mutant était encore plus efficace que le mutant E318K, alors que le simple mutant K182R a montré une activité comparable à celle de la forme sauvage. Ces résultats indiquent que la sumoylation diminue l'activité transcriptionnelle de *MITF.* Les inventeurs ont ensuite vérifié l'effet de la mutation E318K sur des promoteurs physiologiques. Ils se sont concentrés sur MET et HIF1A, deux gènes cibles de *MITF* impliqués dans la tumorigenèse des mélanocytes et du rein. La forme sauvage de *MITF* et le mutant E318K ont montré des activités transcriptionnelles similaires pour le promoteur MET (Figure 2) alors que le mutant E318K présentant une activité transcriptionnelle plus élevée du promoteur HIF1A en comparaison de la forme sauvage. Par conséquent, la mutation p.E318K peut exercer son effet oncogénique via une régulation transcriptionnelle exacerbée de HIF1A.

### Exemple 5

### La mutation E318K confère un phénotype cellulaire moins différentié et plus prolifératif

La production de pigment est un trait de la différentiation des mélanocytes caractérisée par une croissance réduite alors que les cellules faiblement pigmentées corrèlent étroitement avec des phénotypes prolifératifs et moins différentiés. De plus, l'hypoxie et HIF1A soutiennent la survie, la prolifération et la transformation des mélanocytes, et la progression des mélanomes. Par conséquent, les inventeurs ont trouvé que des mélanocytes isolés de biopsies de peau d'un patient portant la mutation germinale étaient moins pigmentés que les mélanocytes de deux donneurs sains ne portant pas la mutation, tous trois de type caucasien (Figure 6). La transcription de *MITF* est modifiée par deux voies de signalisation par des récepteurs, incluant le récepteur de la mélanocortine-1 (MC1R). Enfin, en comparaison de *MITF* sauvage, l'expression de E318K stimulait la croissance des mélanocytes immortalisés (Figure 7A Melan-a), des cellules de mélanomes (Figure 7B, A375) et aussi des cellules de carcinome de rein déficient en VHL (Figure 7C, RCC4). Pris ensemble, les données suggèrent que le mutant de *MITF* E318K confère un avantage de croissance constitutif.

### Matériels et Méthodes

Melarisk est une collection unique de familles enclines aux mélanomes (MELARISK) qui a été initiée en 1985 par l'Institut Gustave Roussy (Pr AVRIL) et l'INSERM ((Florence Demenais, U946), avec une participation des dermatologistes (en particulier, depuis 2005 le CHU Cochin, Pr AVRIL et les Hospices Civils de Lyon, Pr THOMAS) et d'oncogénéticiens. Le matériel biologique est conservé à la Biobanque de susceptibilité au cancer de l'IGR (sang, lymphocytes congelés, lignées cellulaires lymphoblastiques établies par le Généthon, ADN). Les données familiales, démographiques, cliniques et de facteur de risque de mélanome ont été collectées depuis plusieurs années maintenant et sont stockées dans une base de données MySQL dans l'unité INSERM U946.

### Séquençage direct de MITF

Les amorces utilisées pour le séquençage de *MITF* sont données dans le tableau ci-dessous. Le protocole d'amplification consistait en 35 cycles avec des étapes de température à 94°C, 60°C, et 72°C de 30 secondes chacune.

Les produits de PCR ont été séquencés avec la technique de « Big Dye Terminator », version 3.0 (Applied Biosystems, Poster City, Californie) sur des séquenceurs ABI Prism^{©} 3730 Genetic Analyzer (Applied Biosystems, Poster City, Californie).

**Tableau. Séquences des amorces pour le gène MITF**

| Fragment amplifié | Séquence Sens 5' > 3' | Séquence Antisens 5' > 3' | Taille du produit (pb) |
|---|---|---|---|
| Exon *1a_part1* | SEQ ID NO 1 | SEQ ID NO 2 | 377 |
| Exon *1a_part2* | SEQ ID NO 3 | SEQ ID NO 4 | 394 |
| *Exon 1b* | SEQ ID NO 5 | SEQ ID NO 6 | 298 |
| *Exon 1*e | SEQ ID NO 7 | SEQ ID NO 8 | 232 |
| Exon 2 / *Exon 1c* | SEQ ID NO 9 | SEQ ID NO 10 | 396 |
| Exon 1 (M) | SEQ ID NO 11 | SEQ ID NO 12 | 389 |
| Exon 2 | SEQ ID NO 13 | SEQ ID NO 14 | 426 |
| Exon 3 | SEQ ID NO 15 | SEQ ID NO 16 | 271 |
| Exon 4 | SEQ ID NO 17 | SEQ ID NO 18 | 257 |
| Exon 5 | SEQ ID NO 19 | SEQ ID NO 20 | 447 |
| Exon 6a/6b | SEQ ID NO 21 | SEQ ID NO 22 | 280 |
| Exon 7 | SEQ ID NO 23 | SEQ ID NO 24 | 320 |
| Exon 8 | SEQ ID NO 25 | SEQ ID NO 26 | 563 |
| Exon 9 | SEQ ID NO 27 | SEQ ID NO 28 | 544 |

### Génotypage de la mutation MITF E318K par PCR avec une sonde MGB Taqman

Les réactions de PCR ont été faites avec 10 ng d'ADN génomique en présence de 0.2 µmol/L sondes MGB TaqMan soit (5'-VIC-ATC AAG CAA **G**AA CCC G-3'-SEQ ID No 29) qui s'apparie parfaitement avec la séquence de MITF sauvage ou (5'-6-FAM-CAA GCA A**A**A ACC CG-3' - SEQ ID No 30) qui s'apparie parfaitement avec la séquence de MITF qui code la mutation E318K. Les concentrations finales des autres réactifs ont été les suivantes: 1x Universal Master mix (Applied), 0,4 µmol/L d'amorce sens (5'-TGCTCTCCAGATTTGGTGAATCG-3' - SEQ ID No 31), 0,4 µmol/L d'amorce antisens (5'-GGTCTTGGCTGCAGTTCTCAA-3' - SEQ ID No 32). La taille de l'amplicon de PCR est de 67 pb. Le cyclage de PCR a été fait sur un thermocycleur 2720 ABI^{™} comme suit: 95 °C pendant 15 min; 30 cycles de 95 °C pendant 15 s et 60 °C pendant 1 min. La discrimination allélique a été faite par mesure finale de fluorescence sur un 7900HT Fast real Time PCR system ABI^{™} et analysée en utilisant le logiciel SDS v2.3 ABI^{™}. Des échantillons d'ADN soit sauvage soit E318K ont été inclus dans chaque expérience de génotypage comme contrôles. Les échantillons mutés génotypés ont été vérifiés par séquençage direct en utilisant le protocole et les amorces ci-dessus détaillés pour l'exon 9 de *MITF.*

### Plasmides

La construction pCDNA3-Mi de la forme M de *MITF* a été décrite précédemment (7). Les mutations de *MITF,* K182R et/ou E318K, ont été générées avec la méthode QuickChange (Stratagene) en utilisant les amorces sens suivantes et leurs compléments antisens: Mi-K182R 5'-cttcccaacataagaagggagctcacagc-3' (SEQ ID No 33); MI-E318K 5'-ggatcatcaagcaaaaaccagttcttgag-3' (SEQ ID No 34). La présence de mutations a été confirmée par séquençage.

His-SUMO1 et His-HA-SUMO2 étaient des dons de M. A. Dejean et ont été décris dans l'article suivant (8).

### Co-transfections et immunodétection

Des cellules HEK293 cultivées sur des plaques à 6 puits (10⁴ cellules/puit) ont été transfectées avec les plasmides indiqués (2 µg d'ADN total/puit) et du FuGENE 6™ (Roche Applied Science). 48 heures après, les cellules ont été lavées avec du PBS puis lysées à 95 °C dans 1×tampon de charge (41,6 mM Tris, pH 6,8, 1,5% SDS, 6,7% glycérol) et portées à ébullition pendant 5 minutes supplémentaires.

Les protéines ont été séparées par électrophorèse dans un gel 10% SDS-polyacrylamide et transférées sur des membranes PVDF. Les protéines ont été détectées en utilisant l'ECL (Amersham) et des anticorps anti-MITF (Abcam), anti-HA tag (Abcam), anti-SUMO1 (Santa Cruz Biotech) ou anti-ERK2 (Santa Cruz Biotech).

### Tests de mesures d'expression de rapporteurs

Des cellules humaines 501mel et de souris B16 ont été ensemencées dans des plaques à 24 puits (25x10³ cellules/puit) et, les jours suivants, les cellules ont été transfectées de manière transitoire en utilisant 0,3 µg de plasmide rapporteur (pHIF1α et pMet), 0,05 µg de plasmides codant MIT ou de pCDNA2 vide, 2 µl de réactif lipofectamine (Invitrogen) et 0,05 µg pCMVβGa1 pour contrôler la variabilité dans l'efficacité de transfection. Les cellules ont été lysées et testées pour les activités luciférase et β-galactosidase 48 heures après. Les transfections ont été répétées au moins trois fois.

### Immunofluorescence

Les cellules HEK293 ont été ensemencées sur des lamelles de verre (100 x 10³ cellules) dans une plaque à 6 puits et transfectées avec 3 µg de plasmides MITF ou pCDNA3 vide en utilisant 10 µl de lipofectamine. 48 h plus tard, les cellules ont été fixées pendant 10 min avec du paraformaldéhyde 4% dans du PBS, lavées avec du PBS et perméabilisées pendant 2 min avec du Triton X-100 0,1%/albumine sérique bovine 1% (BSA). Ensuite, les échantillons ont été lavés une fois avec du PBS et traités avec NH4Cl 50 mM pendant 2 min, puis lavés 3 fois dans du PBS, et colorés pendant 1 h avec un anticorps anti*-MITF* (Abcam) dans du BSA 1%/PBS. Les échantillons ont alors été lavés 3 fois dans du PBS pendant 5 min et coloré secondairement pendant 1 h avec un anticorps de chèvre anti-souris conjugué au Alexa-488 (Molecular Probes) dans BSA 1%. Les cellules ont été lavées une fois dans du PBS, subies une coloration de contraste avec du 4,6-diamino-2-phénylindole (DAPI), lavées 3 fois dans du PBS, et montées en utilisant Fluromount-G (Southern Biotech, Birmingham AL). Les cellules ont été examinées avec un microscope Zeiss Axiophot équipé avec une illumination épifluorescente.

### Détermination du contenu en mélanine

Approximativement 6 x 10⁶ mélanocytes ont été culotés par centrifugation à 1000 g pendant 5 min et lavées deux fois avec un tampon phosphate. Une partie des culots a été solubilisée dans NaOH 0,5 M pendant 1 h à 80°C et les densités optiques ont été mesurées à 405 nm. L'autre partie a été utilisée pour déterminer le contenu en protéine par un test BCA^{™} (Pierce). Le contenu en mélanine a été corrigé par la concentration en protéines et exprimé en pourcentage de cellules contrôles (100%).

### Test de formation de colonies

Les cellules de mélanome humain A375 et de carcinome rénal humain RCC4 (80000 par puits) ont été transfectées avec un total par puits de 3 µg d'ADN (*MITF* sauvage ou E318K) incluant 10 % de pBABE-puro en utilisant Fugene (Roche). De la puromycine (1 µg/ml) a été ajoutée au milieu 48 h après transfection. 14 jours plus tard, les cellules ont été fixées, colorées avec du cristal violet 0,4% et les plaques ont été photographiées. Les cellules ont également été décolorées avec de l'acide acétique 10 % dans du PBS et l'absorbance à 610 nm, reflétant le nombre de cellules, a été mesurée.

### Immunohistochimie

Après déparaffinage des lames et avoir démasqué les antigènes à chaud dans une solution d'Antigen Unmasking solution (Vector Laboratories), les coupes ont été perméabilisées (0,3% triton / PBS 15min) puis rincées rapidement avec du PBS. Après avoir bloqué les peroxydases endogènes, les coupes ont été saturées pendent 30min dans une solution de PBS / BSA 1% / Sérum de chèvre 5%. Puis elles ont été incubées toute la nuit à 4°C avec le 1^{er} anticorps anti MITF clone C5 (dilution 1/10 ou 1/100) dans une solution de PBS/BSA 1%. Après rinçage dans du PBS, les coupes ont été incubées avec le 2^{éme} anticorps biotinylé dans du PBS/BSA 1% 1h à température ambiante. Les coupes ont ensuite été rincées dans du PBS puis incubées dans une solution Avide/biotine HRP (Kit ABC Elite, Vector Laboratories). Après rinçage dans du PBS, les coupes ont été révélées en présence d'un substrat de peroxydase (kit VIP, Vector Laboratories). Les lames ont finalement été montées au Mountex (Cell Path)

### REFERENCES

1. Levy,C., et al. (2006) Trends Mol.Med., 12, 406-414.
2. Garraway,L.A., et al. (2005) Nature, 436, 117-122.
3. Kido,K., et al. (2009) Cancer Sci. 100, 1863-1869.
4. Larribere, L., et al. (2005) Genes Dev. 19, 1980-1985.
5. Chung, S.Y., et al. (2009) Biosci. Biotechnol. Biochem. 73, 1704-1710.
6. Halder, B., et al. (2009) Carcinogenesis 29, 129-138.
7. Bertolotto C, et al. (1998) J Cell Biol;142:827-35.
8. Bischof O, et al. (2006) Mol Cell;22:783-94.

## Revendications

1. Méthode pour déterminer si un sujet a une prédisposition à ou une susceptibilité de développer un cancer choisi parmi un mélanome cutané malin, un cancer du rein, et des combinaisons de ceux-ci, comprenant déterminer dans un échantillon biologique du sujet si MITF (facteur de transcription associé à la microphtalmie) présente ou non une mutation diminuant ou abolissant la sumoylation de MITF, la présence de cette mutation étant indicative que le sujet a une prédisposition à ou une susceptibilité de développer un tel cancer.

2. Méthode selon la revendication 1, dans laquelle la mutation dans MITF est une substitution d'un résidu lysine et/ou d'un résidu acide glutamique d'un des sites de sumoylation de MITF ou des deux sites.

3. Méthode selon la revendication 2, dans laquelle la mutation est la substitution d'un résidu sélectionné dans le tableau ci-dessous par un quelconque des 19 autres acides aminés.
| Isoforme | SEQ ID No | Ref transcrit | Ref protéine | 1^{er} site de sumoylation | | 2^{ème} site de sumoylation | |
|---|---|---|---|---|---|---|---|
| ISOFORM 1 | 35 | NM_198159 | NP_937802 | K289 | E291 | K417 | E419 |
| ISOFORM 2 | 36 | NM_198177 | NP_937820 | K273 | E275 | K401 | E403 |
| ISOFORM 3 | 37 | NM_006722 | NP_006713 | K288 | E290 | K416 | E418 |
| ISOFORM 4 | 38 | NM_000248 | NP_000239 | K182 | E184 | K316 | E318 |
| ISOFORM 5 | 39 | NM_198158 | NP_937801 | K182 | E184 | K310 | E312 |
| SOFORM 6 | 40 | NM_198178 | NP_937821 | K126 | E128 | K254 | E256 |

4. Méthode selon la revendication 3, dans laquelle la mutation est une substitution de K316 ou E318 de l'isoforme 4 ou du résidu correspondant dans les autres isoformes de MITF.

5. Méthode selon la revendication 4, dans laquelle la mutation est une substitution de E318 de l'isoforme 4 ou du résidu correspondant dans les autres isoformes de MITF, de préférence par un résidu Lysine.

6. Méthode selon l'une quelconque des revendications, dans laquelle la mutation est détectée au niveau protéique ou nucléique.

7. Méthode selon la revendication 6, comprenant
- le séquençage complet ou partiel de MITF, en particulier de la région comprenant le résidu suspecté d'être muté et même uniquement le séquençage de ce résidu particulier ; ou
- la mise en présence de l'ADN génomique, l'ARN ou l'ADNc avec une sonde spécifique de MITF du mutant et éventuellement d'une sonde témoin spécifique de MITF ne présentant pas cette mutation ou de MITF sauvage ; ou
- l'amplification sélective du mutant avec un couple d'amorces dont l'une des amorces s'hybride spécifiquement avec la séquence portant la mutation à détecter.

8. Méthode selon l'une quelconque des revendications 1-6, dans laquelle la mutation est détectée indirectement par la mesure de la sumoylation de MITF, une diminution de la sumoylation par rapport à la protéine MITF sauvage indiquant la présence de la mutation E318K.

9. Méthode selon la revendication 8, dans laquelle la mutation est détectée par la détermination de la localisation cellulaire de MITF en immunohistochimie, une localisation nucléaire étant indicatif de la protéine MITF sauvage alors qu'une localisation nucléaire et cytoplasmique étant indicatif de la protéine MITF mutée E318K.

10. Méthode selon l'une quelconque des revendications, dans laquelle le cancer est la combinaison d'un mélanome cutané malin et d'un cancer du rein.

11. Utilisation *in vitro* de moyens de détection d'une mutation dans MITF diminuant ou abolissant la sumoylation de MITF pour déterminer si un sujet présente ou non une prédisposition à ou une susceptibilité de développer un cancer choisi parmi la liste suivante : un mélanome cutané malin, un cancer du rein, et des combinaisons de ceux-ci.

12. Utilisation selon la revendication 11, dans laquelle les moyens de détection peuvent comprendre ou consister en une sonde spécifique de MITF portant la mutation à détecter, un couple d'amorces permettant l'amplification d'un segment nucléotidique comprenant la mutation à détecter, un couple d'amorces dont l'une d'elles s'hybride spécifiquement avec la séquence portant la mutation à détecter, un anticorps spécifique du mutant à détecter de MITF, des moyens permettant la détection et la mesure de la sumoylation de MITF, des contrôles négatifs permettant de détecter MITF ne présentant pas la mutation à détecter, ou des combinaisons de ceux-ci.

13. Utilisation selon la revendication 11 ou 12, dans laquelle la mutation est la substitution d'un résidu sélectionné dans le tableau ci-dessous par un quelconque des 19 autres acides aminés.
| Isoforme | SEQ ID No | Ref transcrit | Ref protéine | 1^{er} site de sumoylation | | 2^{ème} site de sumoylation | |
|---|---|---|---|---|---|---|---|
| ISOFORM 1 | 35 | NM_198159 | NP_937802 | K289 | E291 | K417 | E419 |
| ISOFORM 2 | 36 | NM_198177 | NP_937820 | K273 | E275 | K401 | E403 |
| ISOFORM 3 | 37 | NM_006722 | NP_006713 | K288 | E290 | K416 | E418 |
| ISOFORM 4 | 38 | NM_000248 | NP_000239 | K182 | E184 | K316 | E318 |
| ISOFORM 5 | 39 | NM_198158 | NP_937801 | K182 | E184 | K310 | E312 |
| SOFORM 6 | 40 | NM_198178 | NP_937821 | K126 | E128 | K254 | E256 |

14. Utilisation selon la revendication 13, dans laquelle la mutation est une substitution de K316 ou E318 de l'isoforme 4 ou du résidu correspondant dans les autres isoformes de MITF.

15. Utilisation selon la revendication 15, dans laquelle la mutation est une substitution de E318 de l'isoforme 4 ou du résidu correspondant dans les autres isoformes de MITF, de préférence par un résidu Lysine.

## Patentansprüche

1. Verfahren zur Bestimmung, ob eine Person eine Prädisposition oder eine Anfälligkeit für die Entwicklung eines Krebses besitzt, der aus einem malignen Hautmelanom, Nierenkrebs und Kombinationen davon ausgewählt ist, umfassend die Bestimmung in einer biologischen Probe der Person, ob MITF (Microphthalmie-assoziierter Transkriptionsfaktor) eine Mutation aufweist oder nicht, die die Sumoylierung von MITF verringert oder aufhebt, wobei das Vorliegen dieser Mutation darauf hinweist, dass die Person eine Prädisposition oder eine Anfälligkeit für die Entwicklung eines derartigen Krebses besitzt.

2. Verfahren gemäß Anspruch 1, wobei die Mutation in MITF eine Substitution eines Lysinrestes und/oder eines Glutaminsäurerestes an einer der Sumoylierungsstellen von MITF oder an beiden Stellen ist.

3. Verfahren gemäß Anspruch 2, wobei die Mutation die Substitution eines aus der nachstehenden Tabelle ausgewählten Restes durch einen beliebigen der 19 anderen Aminosäuren ist.
| Isoform | SEQ ID NR | Ref Transkript | Ref Protein | 1. Sumoylierungsstelle | | 2. Sumoylierungsstelle | |
|---|---|---|---|---|---|---|---|
| ISOFORM 1 | 35 | NM_198159 | NP_937802 | K289 | E291 | K417 | E419 |
| ISOFORM 2 | 36 | NM_198177 | NP_937820 | K273 | E275 | K401 | E403 |
| ISOFORM 3 | 37 | NM_006722 | NP_006713 | K288 | E290 | K416 | E418 |
| ISOFORM 4 | 38 | NM_000248 | NP_000239 | K182 | E184 | K316 | E318 |
| ISOFORM 5 | 39 | NM_198158 | NP_937801 | K182 | E184 | K310 | E312 |
| ISOFORM 6 | 40 | NM_198178 | NP_937821 | K126 | E128 | K254 | E256 |

4. Verfahren gemäß Anspruch 3, wobei die Mutation eine Substitution von K316 oder E318 der Isoform 4 oder des entsprechenden Restes in den anderen MITF-Isoformen ist.

5. Verfahren gemäß Anspruch 4, wobei die Mutation eine Substitution von E318 der Isoform 4 oder des entsprechenden Restes in den anderen MITF-Isoformen, vorzugsweise durch einen Lysinrest, ist.

6. Verfahren gemäß einem der Ansprüche, wobei die Mutation auf Protein- oder Nukleinsäure-Ebene detektiert wird.

7. Verfahren gemäß Anspruch 6, umfassend
- die komplette oder partielle Sequenzierung von MITF, insbesondere der Region, die den vermutlich mutierten Rest umfasst, und sogar lediglich die Sequenzierung dieses speziellen Restes; oder
- das Inkontaktbringen der genomischen DNA, der RNA oder der cDNA mit einer Sonde, die für MITF der Mutante spezifisch ist, und gegebenenfalls einer Kontrollsonde, die für diese Mutation nicht aufweisenden MITF oder für Wildtyp MITF spezifisch ist; oder
- die selektive Amplifikation der Mutante mit einem Primer-Paar, von dem ein Primer mit der Sequenz spezifisch hybridisiert, die die zu detektierende Mutation trägt.

8. Verfahren gemäß einem der Ansprüche 1 bis 6, wobei die Mutation durch die Messung der Sumoylierung von MITF indirekt detektiert wird, wobei eine Verminderung der Sumoylierung im Vergleich zum Wildtyp MITF-Protein auf das Vorliegen der E318K-Mutation hinweist.

9. Verfahren gemäß Anspruch 8, wobei die Mutation durch die immunhistochemische Bestimmung der zellulären Lokalisierung von MITF detektiert wird, wobei eine nukleäre Lokalisierung ein Hinweis auf das Wildtyp MITF-Protein ist, während eine nukleäre und cytoplasmatische Lokalisierung ein Hinweis auf das E318K mutierte MITF-Protein ist.

10. Verfahren gemäß einem der Ansprüche, wobei der Krebs die Kombination eines malignen Hautmelanoms und Nierenkrebs ist.

11. *In vitro* Verwendung von Mitteln zur Detektion einer Mutation in MITF, die die Sumoylierung von MITF vermindert oder aufhebt, um zu bestimmen, ob eine Person eine Prädisposition oder eine Anfälligkeit für die Entwicklung eines Krebses aufweist oder nicht, der aus nachfolgender Liste ausgewählt ist: malignes Hautmelanom, Nierenkrebs oder Kombinationen davon.

12. Verwendung gemäß Anspruch 11, wobei die Mittel zur Detektion eine Sonde, die für MITF spezifisch ist, der die zu detektierende Mutation trägt, ein Primer-Paar, das die Amplifikation eines Nukleotid-Abschnitts erlaubt, der die zu detektierende Mutation umfasst, ein Primer-Paar, von dem ein Primer mit der Sequenz spezifisch hybridisiert, die die zu detektierende Mutation trägt, einen Antikörper, der für die zu detektierende Mutante von MITF spezifisch ist, Mittel, die die Detektion und Messung der Sumoylierung von MITF erlauben, Negativkontrollen, die die Detektion von MITF erlauben, der keine zu detektierende Mutation aufweist, oder Kombinationen davon umfassen oder daraus bestehen können.

13. Verwendung gemäß Anspruch 11 oder 12, wobei die Mutation die Substitution eines aus der nachstehenden Tabelle ausgewählten Restes durch einen beliebigen der 19 anderen Aminosäuren ist.
| Isoform | SEQ ID NR | Ref Transkript | Ref Protein | 1. Sumoylierungsstelle | | 2. Sumoylierungsstelle | |
|---|---|---|---|---|---|---|---|
| ISOFORM 1 | 35 | NM_198159 | NP_937802 | K289 | E291 | K417 | E419 |
| ISOFORM 2 | 36 | NM_198177 | NP_937820 | K273 | E275 | K401 | E403 |
| ISOFORM 3 | 37 | NM_006722 | NP_006713 | K288 | E290 | K416 | E418 |
| ISOFORM 4 | 38 | NM_000248 | NP_000239 | K182 | E184 | K316 | E318 |
| ISOFORM 5 | 39 | NM_198158 | NP_937801 | K182 | E184 | K310 | E312 |
| ISOFORM 6 | 40 | NM_198178 | NP_937821 | K126 | E128 | K254 | E256 |

14. Verfahren gemäß Anspruch 13, wobei die Mutation eine Substitution von K316 oder E318 der Isoform 4 oder des entsprechenden Restes in den anderen MITF-Isoformen ist.

15. Verfahren gemäß Anspruch 14, wobei die Mutation eine Substitution von E318 der Isoform 4 oder des entsprechenden Restes in den anderen MTTF-Isoformen, vorzugsweise durch einen Lysinrest, ist.

## Claims

1. A method for determining whether a subject has a predisposition or a susceptibility to develop a cancer selected from the group consisting of a cutaneous malignant melanoma, a renal cancer, and combinations thereof, comprising determining in a biological sample from the subject if MITF (microphthalmia-associated transcription factor) presents or not a mutation reducing or abolishing the sumoylation of MITF, the presence of said mutation being indicative that the subject has a predisposition or a susceptibility to develop such cancer.

2. The method according to claim 1, wherein the MITF mutation is a substitution of a lysine residue and/or a glutamic acid residue of one of the MITF sumoylation sites or of both sites.

3. The method according to claim 2, wherein the mutation is the substitution of a residue selected from the table below by any of the other 19 amino acids.
| Isoform | SEQ ID No | Transcript ref. | Protein ref. | 1^{st} sumoylation site | | 2^{nd} sumoylation site | |
|---|---|---|---|---|---|---|---|
| ISOFORM 1 | 35 | NM_198159 | NP_937802 | K289 | E291 | K417 | E419 |
| ISOFORM 2 | 36 | NM_198177 | NP_937820 | K273 | E275 | K401 | E403 |
| ISOFORM 3 | 37 | NM_006722 | NP_006713 | K288 | E290 | K416 | E418 |
| ISOFORM 4 | 38 | NM_000248 | NP_000239 | K182 | E184 | K316 | E318 |
| ISOFORM 5 | 39 | NM_198158 | NP_937801 | K182 | E184 | K310 | E312 |
| SOFORM 6 | 40 | NM_198178 | NP_937821 | K126 | E128 | K254 | E256 |

4. The method according to claim 3, wherein the mutation is a substitution of K316 or E318 of isoform 4 or of the corresponding residue in the other MITF isoforms.

5. The method according to claim 4, wherein the mutation is a substitution of E318 of isoform 4 or of the corresponding residue in the other MITF isoforms, preferably by a Lysine residue.

6. The method according to any one of the preceding claims, wherein the mutation is detected at the protein or nucleic level.

7. The method according to claim 6, comprising
- the full or partial sequencing of MITF, in particular of the region comprising the residue suspected to be mutated and even only the sequencing of this particular residue; or
- placing the genomic DNA, RNA or cDNA in the presence of a probe specific for the mutant MITF and optionally a probe specific for MITF not harboring said mutation or wild-type MITF ; or
- selective amplification of the mutant with a primer pair, one of the primers specifically hybridizing with the sequence carrying the mutation to be detected.

8. The method according to any one of claims 1-6, wherein the mutation is indirectly detected by measuring sumoylation of MITF, a reduction of sumoylation relative to the wild-type MITF protein indicating the presence of the E318K mutation.

9. The method according to claim 8, wherein the mutation is detected by determining the cellular localization of MITF by immunohistochemistry, a nuclear localization being indicative of the wild-type MITF protein while a nuclear and cytoplasmic localization being indicative of the E318K mutant MITF protein.

10. The method according to any one of the preceding claims, wherein the cancer is the combination of a cutaneous malignant melanoma and a kidney cancer.

11. *In vitro* use of means for detecting a mutation in MITF reducing or abolishing the sumoylation of MITF for determining whether a subject has or not a predisposition or a susceptibility to develop a cancer selected from the group consisting of a cutaneous malignant melanoma, a renal cancer, and combinations thereof.

12. Use according to claim 11, wherein means for detecting can comprise or consist in a probe specific for MITF harboring the mutation to be detected, a primer pair allowing amplification of a nucleotide segment comprising the mutation to be detected, a pair of primers one of which specifically hybridizes with the sequence carrying the mutation to be detected, an antibody directed against the MITF mutant to be detected, means by which to detect and measure the sumoylation of MITF, negative controls for detecting MITF not carrying the mutation to be detected, or combinations thereof.

13. Use according to claim 11 ou 12, wherein the mutation is the substitution of a residue selected from the table below by any of the other 19 amino acids.
| Isoform | SEQ ID No | Transcript ref. | Protein ref. | 1^{st} sumoylation site | | 2^{nd} sumoylation site | |
|---|---|---|---|---|---|---|---|
| ISOFORM 1 | 35 | NM_198159 | NP_937802 | K289 | E291 | K417 | E419 |
| ISOFORM 2 | 36 | NM_198177 | NP_937820 | K273 | E275 | K401 | E403 |
| ISOFORM 3 | 37 | NM_006722 | NP_006713 | K288 | E290 | K416 | E418 |
| ISOFORM 4 | 38 | NM_000248 | NP_000239 | K182 | E184 | K316 | E318 |
| ISOFORM 5 | 39 | NM_198158 | NP_937801 | K182 | E184 | K310 | E312 |
| SOFORM 6 | 40 | NM_198178 | NP_937821 | K126 | E128 | K254 | E256 |

14. Use according to claim 13, wherein the mutation is a substitution of K316 or E318 of isoform 4 or of the corresponding residue in the other MITF isoforms.

15. Use according to claim 14, wherein the mutation is a substitution of E318 of isoform 4 or of the corresponding residue in the other MITF isoforms, preferably by a Lysine residue.
